# EUROPEAN PATENT SPECIFICATION

(11) **EP 4 364 720 B1**
(45) Date of publication and mention of the grant of the patent: **19.02.2025**
(21) Application number: 22205903.2
(22) Date of filing: 07.11.2022
(51) Int. Cl.: A61K 8/31, A61K 8/362, A61Q 5/08, A61Q 5/10, A61K 8/19, A61K 8/22, A61K 8/44

(54) **OXIDATIVE HAIR TREATMENT COMPOSITIONS**
OXIDATIVE HAARBEHANDLUNGSZUSAMMENSETZUNGEN
COMPOSITIONS OXYDANTES POUR LE TRAITEMENT DES CHEVEUX

(43) Date of publication of application: 08.05.2024
(73) Proprietor: Wella Germany GmbH, 64295 Darmstadt (DE)
(72) Inventor: SENDYUREVA, Viktoriya, 64295 Darmstadt (DE); PRYADILOVA, Olga, 69151 Neckargemünd (DE); HOFFMANN, Björn Timo, 64295 Darmstadt (DE)
(74) Representative: Bandpay & Greuter

(56) References cited:
- JP-A- 2004 168 733
- JP-B2- 6 882 759
- ERLEMANN G ET AL: "PANTHENOL, PHYTANTRIOL, VITAMIN E UND VITAMIN A IN DER KOSMETIK", SOFW JOURNAL, VERLAG FUER CHEMISCHE INDUSTRIE H. ZIOLKOWSKY GMBH, DE, vol. 117, no. 10, 26 June 1991 (1991-06-26), pages 379 - 384, XP000228116, ISSN: 0942-7694

## Description

### FIELD OF THE INVENTION

The presently claimed invention is directed to an oxidative hair treatment composition comprising (A) at least one alkalizing agent, (B) at least one oxidizing agent, (C) at least one saturated acyclic terpane which is a saturated acyclic terpane having 10 to 40 carbon atoms, (D) at least one amino acid selected from the group consisting of alanine, glycine, phenylalanine, leucine, valine, isoleucine; glutamic acid, aspartic acid, citrulline, histidine, arginine, lysine and tyrosine, (E) a cosmetically acceptable aqueous or aqueous-organic medium, and (J) at least one chelant (J); a kit comprising the aforementioned components and a method of treating hair with the oxidative hair treatment composition according to the presently claimed invention.

### BACKGROUND OF THE INVENTION

The problem with oxidatively colouring or blending hair is that the keratin fibres may be damaged by the aggressive substances. In particular, the natural hydrophobicity of the keratin fibres is reduced since the colouring, or lightening agents initially have to rend the hair penetrable so as to develop their effect. The water-repelling action, however, provides natural protection of the hair, and additionally is closely associated with parameters desired by the consumer, such as shine, suppleness, feel and "laying" of the hair.

So as to overcome the aforementioned drawbacks, what are known as pre-treatment agents are on available on the market, which are to protect the hair from aggressive influence. However, these often weigh the hair down or impair the success of the subsequent lightening process or coloration of the hair, and in particular the washing fastness of the colour may be adversely affected by the pre-treatment agent. In addition, numerous pre-treatment agents are known, which are used to attempt to repair damage caused to the air during the oxidative colouring treatment. All of these methods, however, require a multi-stage application process, involving an application of a further hair treatment agent prior to or after colouring. This is frequently perceived as inconvenient by the customer, since the oxidative colouring treatment itself already involves multiple work steps and an exposure time of as much as 60 minutes, making the process very complex.

Permanent, oxidative colouring as well as hair bleaching usually takes place as oxidative hair treatment in the presence of oxidants such as hydrogen peroxide. Unfortunately, the oxidizing agent does not only achieve the desired cosmetic effect, but also damages the structure of the hair keratin. The keratin's own cystine oxidizes to cysteic acid which has a negative effect on the integrity, feel and appearance of the hair fibre. Hair damaged in this way appears dull and brittle.

WO 2018/065428 A1 discloses a rinse-off treatment comprising a mixture of amino acids, wherein the mixture comprises glutamic acid, alanine, and proline.

US 2021/0196606 A1 describe hair treatment compositions comprising amino acids, carboxylic acids, monoethanolamine, at least one surfactant, at least one fatty compound and at least one hair colouring agent.

WO 2021/084084 A1 discloses aqueous oxidizing compositions comprising amino acids and polycarboxylic acids.

JP 6882759 B2 discloses an oxidizing agent composition which is used as a hair dye or a hair bleaching agent comprising squalane.

Erlemann G et al: vol. 117, no. 10, 26 June 1991, pages 379-384 refers to phytantriol, a terpane, for use in the protection of hair.

JP2004168733 A reports on hair dye compositions comprising an alkaline agent, an oxidising agent and amino acids.

There is a need for providing hair treatment compositions that protect hair without negatively influencing the colour result of the oxidative colouring treatment while the oxidative hair treatment takes as little time as possible and takes place directly together with the colouring step.

### SUMMARY OF THE INVENTION

It is an object of the presently claimed invention to provide hair treatment compositions that protect hair without negatively influencing the colour result of the oxidative colouring treatment while the oxidative hair treatment takes as little time as possible and takes place directly together with the colouring step.

Surprisingly it was found that incorporating a specific combination of terpanes and oxidatively stable amino acids into an oxidative hair treatment composition that contains the typical components such as water, alkalizing agents, chelants and oxidizing agents results in considerably improved protection of the hair during the oxidative colouring treatment without impairing the results of the oxidative colouring treatment. This was able to be established, among other things, in that the combing force of hair that was treated with the oxidative hair treatment composition of the presently claimed invention during subsequent combing was considerably decreased in comparison to the combing force of hair that was treated in the absence of squalane and oxidatively stable amino acids.

Accordingly, in one aspect, the presently claimed invention is directed to an oxidative hair treatment composition comprising
(A) at least one alkalizing agent,
(B) at least one oxidizing agent,
(C) at least one saturated acyclic terpane which is a saturated acyclic terpane having 10 to 40 carbon atoms,
(D) at least one amino acid selected from the group consisting of alanine, glycine, phenylalanine, leucine, valine, isoleucine, glutamic acid, aspartic acid, citrulline, histidine, arginine, lysine and tyrosine,
(E) a cosmetically acceptable aqueous or aqueous-organic medium, and
(J) at least one chelant (J) selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, and their salts thereof.

In another aspect, the presently claimed invention is directed to a kit comprising (AA) a tint composition comprising components (A), (C), (D) and (J) as defined herein above and below and (BB) an oxidative composition comprising component (B) and optionally (D) as defined herein above and below.

In yet another aspect, the presently claimed invention is directed to a method of treating hair with the composition according to any of the preceding claims comprises the steps of:
a. providing a tint composition (AA) comprising components (A), and (J) as defined herein above and below,
b. providing an oxidizing composition (BB) comprising component (B) as defined herein above and below,
   wherein components (C) and (D) are present in the tint composition (AA) and/or the oxidizing composition (BB),
c. mixing the tint composition (AA) and the oxidizing composition (BB) to obtain a hair treatment composition according to the presently claimed invention,
d. applying the hair treatment composition onto the hair,
e. leaving the composition on the hair for a period in the range from ≥ 2 to ≤ 50 minutes; and
f. subsequently rinsing the composition from the hair.

### DETAILED DESCRIPTION OF THE INVENTION

The following detailed description is merely exemplary in nature and is not intended to limit the presently claimed invention or the application and uses of the presently claimed invention. Furthermore, there is no intention to be bound by any theory presented in the preceding technical field, background, summary or the following detailed description.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

The term "about", when used in relation to a weight ratio or a weight percentage, means that a specific value should be understood as meaning a range of ± 10 %. For example, the weight proportion of a compound of about 1 %, means a weight proportion ranging from 0.9 to 1.1 %. For example, the weight proportion of a compound of about 2.5 %, means a weight proportion ranging from 2.25 to 2.75 %.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "preferred embodiment" means that a particular feature, structure, or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment but may refer to different embodiments of the presently claimed invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Furthermore, the ranges defined throughout the specification include the end values as well, i.e., a range of 1 to 10 implies that both 1 and 10 are included in the range. For the avoidance of doubt, the applicant shall be entitled to any equivalents according to applicable law.

For the purposes of the presently claimed invention, '% by weight' or 'wt.% 'as used in the presently claimed invention is with respect to the total weight of the oxidative hair treatment composition. Further, the sum of wt.% of all the compounds, as described herein, in the respective components adds up to 100 wt.%.

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

The measurement techniques described hereinabove and herein are well known to a person skilled in the art and therefore do not limit the presently claimed invention.

By "oxidative hair treatment composition", it is meant a ready-to-use composition which can change the colour of hair on which it is applied, and which comprises an alkalizing agent, an oxidizing agent and oxidative dyes.

By "two-component" oxidative hair treatment composition it is meant an oxidative hair treatment composition which is obtained by mixing shortly before use two compositions: a tint composition (AA) and an oxidizing composition (BB). The tint composition (AA) comprises the oxidative primary dye precursors, oxidative coupler dye precursors and the alkalizing agent. The oxidizing composition (BB) comprises the oxidizing agent.

By "composition" it is meant a composition which is mixed by the user with one or more other composition for preparing the ready-to-use oxidative hair treatment composition to be applied to the hair. Hereinafter, the "hair treatment composition" refers to a use/mixed hair treatment composition.

By "user" it is meant the person preparing the hair treatment composition. The user is for example a professional hair stylist working in a salon and is different from the subject on whose hair the composition is applied, or the user is identical to the person on whose hair the composition is applied. The mixing can take place in a bowl or within a bottle or even through a mixer placed between the stored individual compositions and the point at which it is dispensed to be used. While two component oxidative hair treatment compositions are the most commonly found in the market, there are also others which use three or more components. For example, these oxidative hair treatment compositions comprise a tint composition (AA) and a separate alkali composition both of which are mixed in combination with the oxidizing composition (BB) to produce the oxidative hair treatment composition. In such a three-component system, the tint composition (AA) and the alkali composition are considered to be equivalent to the tint composition (AA) of a two-component system.

Alternatively, a tint composition (AA), an anhydrous oxidizer composition and an aqueous composition can be mixed with the anhydrous composition to create the finished mixed oxidative hair treatment composition with the tint composition (AA). In such a three-component system, the anhydrous oxidizer composition and the aqueous composition form the oxidizer composition of the two-component system. An anhydrous dye powder, tablet or granule can also be mixed with an aqueous alkali composition and an oxidizing composition (BB).

Those skilled in the art are aware that there are multiple combinations of the components that can be interchanged to obtain the final mixed hair treatment composition. All these combinations are within the scope of the presently claimed invention which is exemplified in a non-limiting way.

By "lift" (or "lift power") it is meant the amount of lightening caused by the bleaching of the natural hair pigment melanin by using the oxidative hair treatment composition of the presently claimed invention. The amount of lift provided by different hair treatment compositions can be compared by using a human natural dark hair sample (e.g., dark hair of an individual of Chinese descent) and measuring the change of colour achieved following application of the compositions. The change in colour can be measured using well known parameters such as L*a*b* values. A composition can be said to provide a higher lift than another composition, when the resulting L* value or the so-called dL* value (given by L*_{final} - L*ᵢₙᵢₜᵢₐₗ) measured for a given treated sample of dark hair is higher for that composition than for the other composition, using the same experimental conditions. The denomination Level 2 (herein used interchangeably with "demi-permanent" or "tone-on-tone") and Level 3 (herein used interchangeably with "permanent") are commonly used in the hair colour trade to differentiate compositions with medium and high lift.

By "oxidizing agent" it is meant an electron accepting compound suitable for use in hair treatment compositions for removing the natural colour of hair (by destroying the melanin pigment) and reacting with oxidative primary dye precursors to enable the reaction with the oxidative coupler dye precursors to form the colour dye products. The most commonly used oxidizing agent in the art is hydrogen peroxide, however further suitable oxidizing agents that can be used in combination with hydrogen peroxide are described below. Oxidizing agents also include those which produce hydrogen peroxide when contacted with water. For example, sodium percarbonate, sodium perborate and urea peroxide can be used as anhydrous materials, which when contacted with water release hydrogen peroxide. For such oxidizing agents which produce hydrogen peroxide when contacted with water, the wt.% of hydrogen peroxide within mixed oxidative hair treatment composition is considered as the level of b) oxidizing agent.

By "alkalizing agent" it is meant one or more compounds suitable for raising the pH to alkaline level in mixed oxidative hair treatment compositions, in particular to a pH in the range of ≥ 6.5 to ≤ 11. Generally, the most commonly used alkalizing agents in the art are ammonia and monoethanolamine, however the presently claimed invention involves using an alkalizing agent other than ammonia (herein "non-ammonia, non-monoethanolamine" alkalizing agent), in particular the alkanolamine of general formula (I). The alkali agent may be used to keep the pH of the mixed oxidative hair treatment composition to a pH in the range of ≥ 6.5 to ≤ 11.

The below materials are being used in the embodiments of the presently claimed invention. The presently claimed invention is described with non-limiting embodiments. The listed ingredients are the preferred features as well as other preferred but non-limiting features of the presently claimed invention.

In an embodiment, the presently claimed invention is directed to an oxidative hair treatment composition comprising
(A) at least one alkalizing agent,
(B) at least one oxidizing agent,
(C) at least one saturated acyclic terpane which is a saturated acyclic terpane having 10 to 40 carbon atoms,
(D) at least one amino acid selected from the group consisting of alanine, glycine, phenylalanine, leucine, valine, isoleucine, glutamic acid, aspartic acid, citrulline, histidine, arginine, lysine and tyrosine,
(E) a cosmetically acceptable aqueous or aqueous-organic medium, and
(J) at least one chelant (J) selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid and their salts thereof.

In an embodiment, the at least one saturated acyclic terpane (C) is a monoterpane, a sesquiterpane, a diterpane, a sesterterpane, a triterpane or a tetraterpane. In another embodiment, the at least one saturated acyclic terpane (C) is selected from the group consisting of 2,6-dimethyl octane, 2,6,10,15,19-pentamethylleicosane, farnesane, phytane, squalane, isosqualane, neosqualane and lycopane. In yet another embodiment, the at least one saturated acyclic terpane (C) is selected from the group consisting of squalane, isosqualane and neosqualane, preferably the at least one saturated acyclic terpane (C) is squalane.

As used herein, "squalane" refers to an oil and is also referred to as 2,6,10,15,19,23-hexamethyl tetracosane. In one embodiment, the squalane is synthetic squalane which is obtained by chemical synthesis or semisynthetic squalane which is obtained from naturally occurring squalene by reduction. Squalene is present in deep sea fish, such as sharks, or olive oil, rice bran oil, wheat germ oil, sesame oil and cotton seed oil. Squalane can also be obtained from sugar cane.

In a preferred embodiment, the oxidative hair treatment composition comprises squalane derived from a bio-based farnesene or squalane, isosqualane and neosqualane derived from a bio-based farnesene, i.e., the squalane, isosqualane and neosqualane which is produced from bio-based farnesene by catalytic reaction, chemical reaction, thermal reaction, hydrogenation or any combination thereof.

As used herein, the term "bio-based farnesene" refers to farnesene which is biologically produces from microorganisms, in particular genetically modified microorganisms, by fermentation of renewable carbon sources such as sugar. As used herein, "farnesene" refers to α-farnesene, β-farnesene or a mixture thereof. A-farnesene refers to a compound having the following structure: 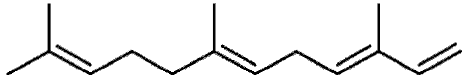 stereoisomer or a mixture of stereoisomers thereof.

B-farnesene refers to a compound having the following structure: 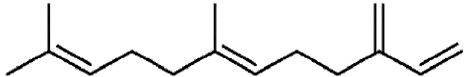 stereoisomer or a mixture thereof.

In one embodiment, β-farnesene is a substantially pure stereoisomer of β-farnesene. In another embodiment, β-farnesene comprises a mixture of stereoisomers, such as cis-trans isomers. In another embodiment, the amount of each of the stereoisomers in the β-farnesene mixture is preferably in the range of ≥ 0.1 wt.% to ≤ 99.9 wt.%, more preferably in the range of ≥ 1.0 wt.% to ≤ 99.0 wt.%, even more preferably in the range of ≥ 5.0 wt.% to ≤ 95.0 wt.%, yet more preferably in the range of ≥ 10 wt.% to ≤ 90 wt.%, most preferably in the range of ≥ 20 wt.% to ≤ 80 wt.%, based on the total weight of the β-farnesene mixture.

In one embodiment, the at least one saturated acyclic terpane (C) has a degree of branching of 2, 3, 4, 5, 6 or 8, preferably the at least one saturated acyclic terpane (C) has a degree of branching of 6.

In yet another embodiment, the at least one component (C) is present in an amount in the range of ≥ 0.1 wt.% to ≤ 5.0 wt.%, preferably in the range of ≥ 0.15 wt.% to ≤ 4.0 wt.%, more preferably in the range of ≥ 0.20 wt.% to ≤ 3.5 wt.%, even more preferably in the range of ≥ 0.2 wt.% to ≤ 3.0 wt.%, most preferably in the range of ≥ 0.25 wt.% to ≤ 2.5 wt.%, based on the total weight of the oxidative hair treatment composition.

The oxidative hair treatment composition comprises at least one amino acid (D) selected from the group consisting of alanine, glycine, phenylalanine, leucine, valine, isoleucine, glutamic acid, aspartic acid, citrulline, histidine, arginine, lysine and tyrosine; preferably the oxidative hair treatment composition comprises at least one amino acid (D) selected from the group consisting of alanine, glycine, valine and arginine; more preferably the at one amino acid (D) is alanine or glycine or valine or arginine. In a preferred embodiment, the oxidative hair treatment composition does not contain other amino acids.

The at least one amino acid (D) which is present in the oxidative hair treatment composition of the presently claimed invention is oxidatively stable in an oxidative hair treatment composition, i.e., the at least one amino acid (D) is not oxidized or only oxidized to a very small degree in an oxidative hair treatment composition by one of a nucleophilic oxidation reaction, an electrophilic oxidation reaction or a radical oxidation reaction.

In one embodiment, the at least one amino acid (D) is obtained from a natural source, more preferably the at least one amino acid (D) is obtained from a plant source.

In another embodiment, the at least one component (D) is present in an amount in the range of ≥ 0.05 wt.% to ≤ 2.0 wt.%, preferably in the range of ≥ 0.1 wt.% to ≤ 1.5 wt.%, more preferably in the range of ≥ 0.1 wt.% to ≤ 1.3 wt.%, even more preferably in the range of ≥ 0.1 wt.% to ≤ 1.0 wt.%, based on the total weight of the oxidative hair treatment composition.

In an embodiment, the at least one alkalizing agent (A) is selected from the group consisting of ammonia and alkanolamines. In one embodiment, the alkanolamines are selected from the group consisting of monoethanolamine, propanolamine, isopropanolamine, 2-amino-2-methyl-1-propanol, 2-amino-1-propanol, 2-amino-1-butanol, 1-amino-2-propanol, 3-amino-1-propanol, 2-aminopropane-1,3-diol, 1-amino-2-butanol, 4-amino-2-butanol, 4-amino-1-butanol, 3-amino-1-butanol, 3-amino-2-butanol, 2-amino-1-butanol, dimethyl glucamine, 2,5-diaminocyclohexane-1,4-diol, 2-aminocycohexan-1-ol, 2,4,6-triaminocyclohexane-1,3,5-triol, 2-amino-2-methyl-1,3-popanediol, 2-amino-1-hydroxylmethyl-1,3-propanediol and 2-dimethylamino-2-methyl-1-propanol. In a preferred embodiment, the at least one alkalizing agent (A) is selected from the group consisting of ammonia, monoethanolamine, propanolamine, isopropanolamine and 2-amino-1-propanol; more preferably the at least one alkalizing agent (A) is monoethanolamine and/or ammonia.

In another embodiment, the at least one alkalizing agent (A) is a combination of at least one source of carbonate ions or hydrogen carbonate ions or carbamate ions and at least one source of an alkalizing agent. In a preferred embodiment, the at least one source of carbonate ions or hydrogen carbonate ions or carbamate ions is selected from the group consisting of sodium, potassium, guanidine, arginine, lithium, calcium, magnesium, barium, ammonium salts of carbonate, carbamate and hydrogen carbonate ions and the at least one source of an alkalizing agent is selected from the group consisting of ammonium chloride, ammonium sulfate, ammonium persulfate, ammonium nitrate, ammonium phosphate, ammonium acetate, ammonium hydroxide and ammonia.

In a preferred embodiment, the at least one source of carbonate ions or hydrogen carbonate ions or carbamate ions is selected from the group consisting of sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate and ammonium hydrogen carbonate. Percarbonate salts may also be utilized to provide both the source of carbonate ions and oxidizing agent.

In a preferred embodiment, the at least one component (A) is present in an amount in the range of ≥ 0.05 wt.% to ≤ 8.0 wt.%, preferably in an amount in the range of ≥ 0.05 wt.% to ≤ 7.0 wt.%, more preferably in an amount in the range of ≥ 0.1 wt.% to ≤ 6.5 wt.%, most preferably in an amount in the range of ≥ 0.1 wt.% to ≤ 5.0 wt.%, based on the total weight of the oxidative hair treatment composition.

The oxidizing agents are preferably water-soluble inorganic peroxide materials which are capable of yielding hydrogen peroxide in an aqueous solution.

In one embodiment of the presently claimed invention, the oxidizing are selected from the group consisting of hydrogen peroxide, inorganic alkali metal peroxides (such as sodium periodate and sodium peroxide), organic peroxides (such as urea peroxide, melamine peroxide), inorganic perhydrate salt bleaching compounds (such as the alkali metal salts of perborates, percarbonates, perphosphates, persilicates, persulphates), and mixtures thereof; preferably from the group consisting of hydrogen peroxide, persulphates, and mixtures thereof; most preferably the oxidizing agent is hydrogen peroxide.

"Water-soluble," as defined herein, means that in standard condition at least 0.1 g, 1 g, or 10 g of the oxidizing agent can be dissolved in 1 litre of deionized water.

In one embodiment, the component b) is preferably present in an amount in the range of ≥ 0.6 wt.% to ≤ 30.0 wt.%, more preferably in an amount in the range from of ≥ 0.7 wt.% to ≤ 25.0 wt.%, more preferably in an amount in the range from of ≥ 0.75 wt.% to ≤ 20.0 wt.%, based on the total weight of the oxidative hair treatment composition.

The oxidizing agents can be provided in aqueous solution or as a powder which is dissolved prior to use.

In an embodiment, the oxidative hair treatment composition comprises the cosmetically acceptable aqueous or aqueous-organic medium (E) is present in an amount in the range of ≥ 30.0 to ≤ 95.0 wt.%, preferably in an amount in the range of ≥ 35.0 to ≤ 92.0 wt.%, more preferably in an amount in the range of ≥ 40.0 to ≤ 90.0 wt.%, based on the total weight of the oxidative hair treatment composition. The cosmetically acceptable aqueous medium (E) consists of water. The cosmetically acceptable aqueous-organic medium (E) consists of water and a water miscible organic solvent such as an alcohol. The cosmetically acceptable aqueous-organic medium (E) preferably comprises water and a monoalcohol such as ethanol and isopropanol and/or a polyalcohol such as propylene glycol, hexylene glycol, glycerin and propane diol.

In one embodiment of the presently claimed invention, the oxidative primary dye precursors (F) are selected from of the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof.

In one embodiment of the presently claimed invention, the oxidative coupler dye precursors (G) are selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotolueneµ, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate, resveratrol and salts thereof.

These and other primary dye precursors (F) and coupler dye precursors (G) may be used in different combination to achieve the nuance sought, as is known in the art.

Direct dyes (I) may also be incorporated in any of components of the presently claimed invention, in particular in the tint composition (AA). The compositions of the presently claimed invention may also comprise compatible direct dyes (I), in an amount that is sufficient to provide additional colouring, particularly with regard to intensity. Typically, such an amount will range from 0.005% wt.% to 4.0 wt.%, based on the total weight of the composition. When the composition is obtained by mixing a tint composition (AA) and an oxidizing composition (BB), the direct dyes are usually incorporated in the tint composition (AA).

In one embodiment of the presently claimed invention, direct dyes (I) are selected from the group consisting of Acid Yellow 1, Disperse Red 17, Basic Brown 17, Acid Yellow 9, Pigment Red 49, Acid Yellow 11, Acid Black 1, 4-Nitro-1,2-phenylenediamine, Picramic acid, HC Red 13, N,N'-Bis(2-hydroxyethyl)-2-nitro-p-phenylenediamine, HC Yellow 5, HC Red 7, HC Blue 2, HC Yellow 4, HC Yellow 3, HC Blue 1, HC Yellow 2, HC Orange 1, HC Red 1, HC Red 3, 4-Amino-3-nitrophenol, 2-Hydroxyethylamino-5-nitroanisole, 3-Nitro-p-hydroxyethylaminophenol, 3-Methylamino-4-nitrophenoxyethanol, 2-Nitro-5-glyceryl methylaniline, HC Violet 1, HC Orange 2, HC Orange 3, HC Yellow 9, 4-Nitrophenyl aminoethylurea, HC Red 10, HC Red 11, 2-Hydroxyethyl picramic acid, HC Blue 12, HC Yellow 6, Hydroxyethyl-2-nitro-p-toluidine, HC Yellow 12, HC Blue 11, HC Blue 10, HC Blue 6, HC Yellow 7, HC Yellow 10, HC Blue 9, HC Blue 13, 2-Chloro-6-(ethylamino)-4-nitrophenol, 6-Nitro-2,5-pyridinediamine, HC Violet 2, 2-Amino-6-Chloro-4-Nitrophenol, 4-Hydroxypropylamino-3-nitrophenol, Acid Blue 7, HC Yellow 13, HC Red 14, HC Yellow 15, HC Yellow 14, 2,6-Diamino-3-((pyridin-3-yl)azo)pyridine, Basic Orange 69, HC Red 16, Basic violet 2, Basic Red 51, Basic Yellow 87, Basic Orange 31, HC Blue 16, HC red 17, HC Yellow 17, HC blue 18, HC Yellow 16, HC Red 18, HC Orange 6, Basic Orange 1, Basic Red 76, Basic Brown 16, Basic Yellow 57, CI14700, Acid Red 14, Acid Orange 7, Acid Red 88, FD&C Yellow 6, Acid Red 27, Acid Orange 10, Acid Red 33, Acid red 155, Acid Yellow 23, Food Black 2, CI 28440, HC Brown 2, Acid Blue 1, Acid Blue 3, Acid Blue 9, Acid Violet 49, CI 42735, Basic Blue 26, Acid violet 9, Acid Red 92, Acid Yellow 3, Beta-Carotene, CI 50420, Basic Blue 99, 1-Amino-4-hydroxy-9,10-anthracendion, CI 60725, Acid violet 43, Disperse Violet 1, Acid blue 62, Copper phthalocyanine, Carbon black, Disperse Black 9, Lycopene, Hydroxyanthraquinoneaminopropyl methyl morpholinium methosulfate, HC Blue 8, HC Green 1, Curry red, 2-Hydroxy-1,4-naphthoquinone, CI 77289, Solvent Green 7, Lawsonia Inermis Cera, CI 73000, Indigofera tinctoria, Carmines, HC Blue 14, Acid Red 18, CI 42053, Acid Red 52, Acid Green 25, Disperse Blue 377, Pigment Red 57, HC Blue 15, Tetrabromphenol Blue, Cationic Blue 347, Bromthymol blue sodium, Anthocyanins, Chlorophyllin-Copper complex, Annatto, Natural Green 3, Betanin, Capsanthin, Basic Yellow 29 and combinations thereof.

In an embodiment, the oxidative hair treatment composition according to the presently claimed invention comprises at least one buffering agent (H) which is selected from the group consisting of buffering alkali compounds and buffering acidic compounds. In one embodiment, the buffering alkali compounds are selected from the group consisting of alkali metal salts and the buffering acidic compounds are selected from the group consisting of sulfurous acid, sulphuric acid, hydrochloric acid, hyponitrous acid, nitrous acid, nitric acid, phosphoric acid, phosphorous acid, citric acid, and malic acid. In another embodiment, the at least one component (H) is preferably selected from the group consisting of disodium phosphate, potassium chloride, phosphoric acid, citric acid, malic acid, hydrochloric acid, hyponitrous acid, and mixtures thereof, more preferably the at least one component (H) is citric acid.

In another embodiment, the at least one component (H) is a mixture of a buffering alkali composition and a buffering acidic composition, wherein the buffering alkali composition is disodium phosphate, and the buffering acidic composition is phosphoric acid.

In one embodiment, the at least one component (H) is present in an amount in the range of ≥ 0.05 wt.% to ≤ 4.0 wt.%, based on the total weight of the oxidative hair treatment composition.

In one embodiment, the oxidative hair treatment composition according to the presently claimed invention has a pH in the range from ≥ 6.0 to ≤ 11.0; more preferably a pH in the range from ≥ 6.5 to ≤ 11.0.

Chelants (J) are preferably used prior to application of a oxidative hair treatment composition to remove and/or bind metal ions which are bonded to hair. For example, US 5,635,167 discloses a process for the removal of exogenous metal ions that are attached to hair. The treatment comprises contacting hair with a blend of chelating agents (selected from the group consisting of amino acid chelating agents, polyphosphate chelating agents and phosphonate chelating agents) at a pH of between 4 and 9 and at a concentration of between 4% to 25% by weight prior to colouring.

As already disclosed in WO 02/089754, among the usual ingredients encountered within an oxidative hair treatment composition, the presence of a chelant (J) such EDDS and its salts thereof may be beneficial in several ways, as chelants can reduce hair damage caused by oxidizing agents.

In a further embodiment of the presently claimed invention, the at least one chelant (J) is selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, salts thereof, derivatives thereof, and mixtures thereof.

In one embodiment of the presently claimed invention, the at least one chelant (J) is EDDS, more preferably is (S,S)-EDDS and their salts thereof.

In one embodiment, an additional chelant selected from the group consisting of sodium phytate, phytic acid, ethylglycine-N,H-diacetic acid, diethylenetriamine penta(methylene phosphonic acid), diethylenetriamine pentaacetate, pentetic acid and hydroxyethylethylenediaminetriacetic acid is present.

In a further embodiment of the presently claimed invention, the oxidative hair treatment composition comprises at least one chelant (J) in an amount in the range from ≥ 0.20 wt.% to ≤ 2.0 wt.%, based on the total weight of the oxidative hair treatment composition.

In one embodiment of the presently claimed invention, the oxidative hair treatment composition preferably comprises at least one fatty substance (K) that does not contain any free carboxylic acid groups, i.e. free of -C(=O)-OH groups and -C(=O)-O⁻. The term "fatty substance" means an organic compound that is insoluble in water at room temperature (25°C) and at atmospheric pressure (≤ 15 mg/L water, preferably ≤ 5 mg/L water). In addition, under the same temperature and pressure conditions, the fatty substances are soluble in organic solvents such as chloroform, ethanol or benzene. In a preferred embodiment, the at least one fatty substance (K) is chemically different from the at least one saturated acyclic terpane which is a saturated acyclic terpane having 10 to 40 carbon atoms.

In a preferred embodiment of the presently claimed invention, the fatty substances (K) are selected from the group consisting of liquid hydrocarbons, non-silicone oils of animal, plant, mineral or synthetic origin, fatty alcohols, fatty acids, fatty acid esters and/or fatty alcohol esters, non-silicone waxes, and silicones or mixtures thereof.

The liquid hydrocarbons are chemically different from the at least one saturated acyclic terpane which is a saturated acyclic terpane having 10 to 40 carbon atoms.

More particularly, the liquid hydrocarbons are selected from the group consisting of:
- hexane, undecane, dodecane, tridecane, and isoparaffins, such as isohexadecane, isododecane and isodecane,
- linear or branched hydrocarbons of mineral, animal or synthetic origin with more than 16 carbon atoms, such as volatile or non-volatile liquid paraffins and derivatives thereof, petroleum jelly, liquid petroleum jelly, polydecenes and hydrogenated polyisobutylene such as Parleam.

In a preferred embodiment of the presently claimed invention, the liquid hydrocarbons are selected from the group consisting of volatile or non-volatile liquid paraffins, and liquid petroleum jelly.

The compositions of the presently claimed invention preferably comprises an amount of fatty substances (K) that do not contain any free carboxylic acid groups of ≥ 2.0 wt.%, more preferably ≥ 10.0 wt.%, even more preferably ≥ 20 wt.%, most preferably ≥ 30 wt.%, based on the total weight of the oxidative hair treatment composition.

In a further embodiment of the presently claimed invention, the oxidative hair treatment composition comprises fatty substances (K) in an amount in the range from ≥ 2.0 wt.% to ≤ 70.0 wt.%, based on the total weight of the oxidative hair treatment composition.

In one embodiment of the presently claimed invention, a creamy carrier for the tint composition (AA) preferably comprises from ≥ 10.0 wt.% to ≤ 30 wt.% of at least one fatty alcohol with 10 to 24 carbon atoms; and/or one or more of ≥ 0.2 wt.% to ≤ 6.0 wt.% of at least one diester of formula: R¹-CO-O-(CH2-CH2-O)ₙ-CO-R², where n is 1, 2 or 3, and R1 and R2 are the same or different alkyl radicals with 12 to 20 carbon atoms; and/or one or more of ≥ 0.5 wt.% glycerine fatty acid ester with 10 to 24 carbon atoms; and/or one or more of ≥ 0.1 wt.% to ≤ 10 wt.% of non-ionic and/or anionic and/or ampholytic emulsifiers, based on the total weight of the tint composition (AA).

Lower levels of fatty alcohol can also be used in this chassis, if a less thick composition is desired, for example a level of from ≥ 2.0 % wt.% to ≤ 10 wt.% of at least one fatty alcohol with 10 to 24 carbon atoms can be used.

The formulations disclosed in WO 98/11863 A2 may also be used as a reference for the presently claimed invention. The formulations disclosed in this reference document contain a beeswax-protein hydrolysate-and/or amino acid association, which however may or may not be present in the tint component of the presently claimed invention.

In one embodiment of the presently claimed invention, the oxidative hair treatment composition may be a three-component system, as exemplified in US 2010/0223739 A2, in which case the aqueous cosmetic composition comprising at least one fatty substance (K) and at least one surfactant (M) as defined in this document may be considered as a third composition.

The oxidative hair treatment composition s of the presently claimed invention preferably comprises a thickener (L), in particular a polymeric thickener in an amount that is sufficient to impart a viscosity to the composition that allows for its ready application to hair without unduly dripping off the hair, as is known in the art. Typically, such an amount will be at least about 0.1 wt.%, in some embodiments, at least about 0.5 wt.%, in other embodiments, at least about 1.0 wt.%, based on the total weight of the oxidative hair treatment composition .

Examples of commonly used associative polymeric thickeners are sold under the tradename Aculyn-22 and Aculyn-33 by the company Rohm & Haas, Permulen TR1, Carbopol 2020, Carbopol Ultrez-21 by the company Noveon, and Structure 2001 and Structure 3001 by the company National Starch. Other suitable polymers include polyether polyurethanes, for example Aculyn-44 and Aculyn-46 by the company Rohm and Haas. Another suitable associative polymer is cellulose modified with groups comprising at least one C8 - C30 fatty chain, such as the product Natrosol Plus Grade 330 CS sold by the company Aqualon.

In one embodiment of the presently claimed invention the oxidative hair treatment composition comprises salt tolerant thickeners (L), including but not limited to: xanthan, guar, hydroxypropyl guar, scleroglucan, methyl cellulose, ethyl cellulose (available as AQUACOTE^{®}, hydroxyethyl cellulose (NATROSOL^{®}), carboxymethyl cellulose, hydroxypropylmethyl cellulose, microcrystalline cellulose, hydroxybutylmethyl cellulose, hydroxypropyl cellulose (available as KLUCEL^{®}), hydroxyethyl ethyl cellulose, cetyl hydroxyethyl cellulose (available as NATROSOL^{®} Plus 330), N-vinylpyrrolidone (available as POVIDONE^{®}), diutan gum, Acrylates/Ceteth-20 Itaconate Copolymer (available as STRUCTURE^{®} 3001), hydroxypropyl starch phosphate (available as STRUCTURE^{®} ZEA), polyethoxylated urethanes or polycarbamyl polyglycol ester (e.g. PEG-150/Decyl/SMDI copolymer (e.g. ACULYN^{®} 44), PEG-150/Stearyl/SMDI copolymer (available as ACULYN^{®} 46), trihydroxystearin (available as THIXCIN^{®}), acrylates copolymer (e.g. available as ACULYN^{®} 33) or hydrophobically modified acrylate copolymers (e.g. Acrylates / Steareth-20 Methacrylate Copolymer (available as ACULYN^{®} 22), acrylates/steareth-20 methacrylate crosspolymer (available as ACULYN^{®} 88), acrylates/vinyl neodecanoate crosspolymer (available as ACULYN^{®} 38), acrylates/beheneth-25 methacrylate copolymer (available as ACULYN^{®} 28), acrylates/C 10-30 alkyl acrylate crosspolymer (available as Carbopol^{®} ETD 2020), non-ionic amphophilic polymers comprising at least one fatty chain and at least one hydrophilic unit selected from polyether urethanes comprising at least one fatty chain,.

In one embodiment of the presently claimed invention, the oxidative hair treatment composition comprises at least one mineral thickener (L) selected from organophilic clays, fumed silicas, or mixtures thereof. The at least one mineral thickener is present in an amount ranging from ≥ 1.0 to ≤ 30 wt.%, based on the total weight of the oxidative hair treatment composition.

In one embodiment of the presently claimed invention, the organic thickener (L) is selected from cellulose-based thickeners (hydroxyethycellulose, hydroxypropyl cellulose, carboxymethylcellulose), guar gum and derivatives thereof (hydroxypropyl guar), gums of microbial origin (xanthan gum, scleroglucan gum), crosslinked homopolymers of acrylic acid or of acrylamido-propanesulfonic acid, and cellulose-based thickeners, such as hydroxyethylcellulose. The at least one organic thickener is present in an amount ranging from ≥ 0.1 to ≤ 20 wt.%, based on the total weight of the oxidative hair treatment composition.

In one embodiment of the presently claimed invention, the oxidative hair treatment composition preferably comprises a gel network thickener system (L). The gel network thickener system (L) is typically provided in the tint composition (AA) and subsequently mixed with the oxidizing composition (BB) whilst retaining the gel like network system in the resultant mixed oxidative hair treatment composition. Such gel network thickener systems comprise a surfactant and a fatty alcohol.

In one embodiment of the presently claimed invention, the gel network system (L) preferably comprises a linear or branched C14 to C30 fatty alcohol, preferably selected from the group consisting of cetyl alcohol, stearyl alcohol, cetostearyl alcohol or behenyl alcohols or mixtures thereof. According to the presently claimed invention the tint composition (AA) preferably comprises from ≥ 1.0 wt.% to ≤ 18.0 wt.%, more preferably from ≥ 2.0 wt.% to ≤ 12.0 wt.% of fatty alcohol.

In one embodiment of the presently claimed invention, the gel network system(L) preferably comprises an ionic, cationic or non-ionic surfactant or amphiphile.

The gel network thickener system (L) preferably comprises at least one anionic surfactant selected from C8 to C30 alkyl phosphate, C8 to C30 alkyl ether phosphate or mixtures thereof, and a C14 to C30 fatty alcohol. Other anionic gel network forming surfactants may also be used.

The gel network thickener system (L) preferably comprises at least one non-ionic surfactant selected from the group consisting of fatty alcohol polyoxyalkylene esters, alkyl polyoxyalkylene ethers which are derived from C7-C30-fatty alcohols, alkylaryl alcohol polyoxyethylene ethers, alkoxylated animal and/or plant fats and/or oils, glycerol esters, alkylphenol alkoxylates, fatty amine alkoxylates, fatty acid amide and fatty acid diethanolamide alkoxylates, ethoxylates thereof, sugar surfactants, sorbitol esters, polyoxyethylene sorbitan fatty acid esters, alkyl polyglycosides, N-alkylgluconamides, alkyl methyl sulfoxides and alkyl dimethyl phosphine oxides.

Representative examples of anionic surfactants include salts (such as alkaline salts, for example, sodium salts, ammonium salts, amine salts, amino alcohol salts and magnesium salts) of the following compounds: alkyl ether carboxylates, alkyl ether sulphates, alkyl glyceryl sulphonates, alkylamido ether sulphates, alkylarylpolyether sulphates, alkyl monoglyceride sulphates, alkyl ether sulphonates, alkylamide sulphonates; alkyl succinates, alkyl sulphosuccinates, alkyl ether sulphosuccinates, alkylamide sulphosuccinates; alkyl sulphosuccinamates; alkyl sulphoacetates; alkyl ether phosphates; acyl sarcosinates, N-acyl methylaminopropionate; acyl isethionates, N-acyl taurates; acyl lactylates; carboxyalkyl ether of alkyl polyglucosides; alkyl lecithin derivatives. The alkyl or acyl radical of all of these various compounds, for example, comprises from about 8 to 30 carbon atoms, and the aryl radical, for example, is chosen from phenyl and benzyl groups.

Anionic surfactants include alkyl ether phosphates, alkyl ether sulphates, alkyl glyceryl sulphonates, N-acyl sarcosinates, N-acyl taurates, acyl lactylates and carboxyalkyl ether of alkyl polyglucosides. Yet more preferable surfactants are selected from alkyl ether phosphates having an average 1 to 20, 1 to 10 or 1 to 3 ethylene oxide units.

The gel network thickener system (L) preferably comprises at least one cationic surfactant selected from quaternary ammonium salts or amido-amines having at least one fatty chain containing at least about 20 carbon atoms and mixture thereof.

The quaternary ammonium salts have the general formula N⁺(R¹R²R³R⁴)X⁻: wherein, R¹ is selected from linear and branched radicals comprising about 20 to 30 carbon atoms, R² is selected from linear and branched radicals comprising about 20 to 30 carbon atoms or the same group as radicals R³ to R⁴, the radicals R³ to R⁴, which can be identical or different, are selected from linear and branched aliphatic radicals comprising from about 1 to 4 carbon atoms, and aromatic radicals such as aryl and alkylaryl, the aliphatic radicals can comprise at least one hetero atom such as oxygen, nitrogen, sulphur and halogens, the aliphatic radicals are chosen, for example, from alkyl, alkoxy and alkylamide radicals, and wherein X⁻ is an anion selected from halides, such as chloride, bromide and iodide) (C₂-C₆)alkyl sulphates, such as methyl sulphate, phosphates, alkyl and alkylaryl sulphonates, and anions derived from organic acids, such as acetate and lactate. The cationic surfactant is, for example, a behentrimonium chloride, behenamidopropyltrimonium methosulfate, stearamidopropyltrimonium chloride, arachidtrimonium chloride and mixtures thereof.

The amido-amine have the general formula R'¹-CONH(CH₂)ₙNR'²R'³:
wherein, R'¹ is selected from linear and branched radicals comprising about 20 to 30 carbon atoms, the radicals R'² and R'³, which can be identical or different, are selected from hydrogen, linear and branched aliphatic radicals comprising from about 1 to 4 carbon atoms, and aromatic radicals such as aryl and alkylaryl, the aliphatic radicals can comprise at least one hetero atom such as oxygen, nitrogen, sulphur and halogens, the aliphatic radicals are chosen, for example, from alkyl, alkoxy and alkylamide radicals, and wherein n is integer from 1 to 4. The amido-amine can be selected from, for example, behenamidopropyldimethylamine, behenamidopropyldiethylamine, behenamidoethyldiethylamine, behenamidoethyldimethylamine, arachidamidopropyldimethylamine, arachidamido-propyidiethylamine, arachidamidoethyidiethylamine, arachidamidoethyidimethylamine, and mixtures thereof.

In one preferred embodiment of the presently claimed invention, the surfactant which is used to form the gel network systems is selected from the group consisting of ceteareth-25, steareth-20, steareth-100, steareth-150, steareth-200 and mixtures thereof. These surfactants act as a co- emulsifier and stabilizer of the gel network system.

Those skilled in the art will recognize that gel network thickener systems usually have a complex structure of networked lamellar bi-layers and / or vesicles and sometimes crystals. These systems usually have creamy appearance and feel and are thus particularly desirable.

In one embodiment of the presently claimed invention, the hair colouring formulation may further comprise at least one anionic, cationic, nonionic, amphoteric, or zwitterionic polymer or mixtures thereof; antioxidants; penetrants; sequestrants; fragrances; dispersants; film-forming agents; ceramides; preserving agents; and opacifiers.

In a preferred embodiment of the presently claimed invention the suitable non-associative cross-linked polycarboxylic polymers for use herein can be selected, for example, from: (i) cross-linked acrylic acid homopolymers; or (ii) copolymers of acrylic or (meth)acrylic acid and of C1-C6 alkyl acrylate or (meth)acrylate. Such polymers are sold under the names Carbopol 980, 981, 954, 2984, 5984 by the company Noveon or Synthalen M, Synthalen L and Synthalen K by the company 3V Sigma, or Aculyn-33 by the company Rohm and Haas.

Polysaccharides may also be used, for example, glucans, modified and unmodified starches, amylose, amylopectin, glycogen, dextrans, celluloses and derivatives thereof (methylcelluloses, hydroxyalkylcellulose, ethyl hydroxyethylcellulose, and carboxymethylcelluloses), mannans, xylans, lignins, arabans, galactans, galacturonans, chitin, chitosans, glucuronoxylans, arabinoxylans, xyloglucans, glucomannans, pectic acids and pectins, alginic acid and alginates, arabinogalactans, carrageenans, agars, glycosaminoglucans, gum arabics, gum tragacanths, ghatti gums, karaya gums, carob gums, galactomannans, such as guar gums, and nonionic derivatives thereof (hydroxypropyl guar) and bio-polysaccharides, such as xanthan gums, gellan gums, welan gums, scleroglucans, succinoglycans and mixtures thereof. Suitable polysaccharides are described in "Encyclopedia of Chemical Technology", Kirk-Othmer, Third Edition, 1982, volume 3, pp. 896-900, and volume 15, pp. 439-458, in "Polymers in Nature" by E. A. MacGregor and C. T. Greenwood, published by John Wiley & Sons, Chapter 6, pp. 240-328,1980, and in "Industrial Gums-Polysaccharides and their Derivatives", edited by Roy L. Whistler, Second Edition, published by Academic Press Inc.

The oxidizing composition (BB) of the presently claimed invention may be based on the same or similar (i.e. having the same ingredients but possibly at different level) formulation chassis as the tint component.

In one embodiment of the presently claimed invention, the oxidative hair treatment composition of the invention is obtained by mixing the tint composition (AA) and oxidizing composition (BB). The tint composition (AA) comprises at least one alkalizing agent (A) and a mixture of oxidative dyes. The oxidizing composition (BB) comprises the oxidizing agent. Such two component systems are commonly used in the art and each component is formulated so that the resulting mixture is a oxidative hair treatment composition according to the presently claimed invention.

In one embodiment of the presently claimed invention, before use, the tint (AA) and oxidizing composition (BB) used in the invention are normally packaged separately from one another. The compositions may be packaged in separate primary packages such as plastic bottle, sachet or tube. The components, in particular each component of a two-component composition, may however be packaged separately but within a common secondary package such as a carton or in different compartment of an aerosol or foam bottle, as in known in the trade. A conditioning composition, which can be applied after rinsing of the oxidative hair treatment composition, may also be packaged in such secondary package.

In one embodiment of the presently claimed invention, the oxidative hair treatment composition is provided in the form of a 3 component kit comprising the tint (AA) and the oxidizing composition (BB) which is provided in separate packages, whereby one package contains the source of an alkalizing agent such as ammonium persulfate (the so-called "bleach) and the other package contains an oxidizing agent such hydrogen peroxide (the so-called "developer").

In an embodiment, the presently claimed invention is directed to an oxidative hair treatment composition comprising
(A) at least one alkalizing agent selected from the group consisting of ammonia, monoethanolamine, propanolamine, isopropanolamine and 2-amino-1-propanol in an amount in the range of ≥ 0.05 wt.% to ≤ 8.0 wt.%,
(B) at least one oxidizing agent selected from the group consisting of hydrogen peroxide, inorganic alkali metal peroxides, inorganic perhydrate salts and organic peroxides in an amount in the range of ≥ 0.6 wt.% to ≤ 8.0 wt.%,
(C) squalane in an amount in the range of ≥ 0.1 wt.% to ≤ 5.0 wt.%,
(D) at least one amino acid selected from the group consisting of alanine, glycine, phenylalanine, leucine, valine, isoleucine and arginine in an amount in the range of ≥ 0.05 wt.% to ≤ 2.0 wt.%,
(E) a cosmetically acceptable aqueous or aqueous-organic medium, and
(J) ethylenediamine-N,N'-disuccinic acid (EDDS), and its salts thereof, in an amount from ≥ 0.20 wt.% to ≤ 2.0 wt.%,
whereby the amount in wt.% is in each case based on the total weight of the oxidative hair care composition.

In an embodiment, the presently claimed invention is directed to an oxidative hair treatment composition comprising
(A) at least one alkalizing agent selected from the group consisting of ammonia, monoethanolamine, propanolamine, isopropanolamine and 2-amino-1-propanol in an amount in the range of ≥ 0.1 wt.% to ≤ 5.0 wt.%,
(B) at least one oxidizing agent selected from the group consisting of hydrogen peroxide, inorganic alkali metal peroxides, inorganic perhydrate salts and organic peroxides in an amount in the range of ≥ 0.75 wt.% to ≤ 6.0 wt.%,
(C) squalane in an amount in the range of ≥ 0.2 wt.% to ≤ 3.0 wt.%,
(D) at least one amino acid selected from the group consisting of alanine, glycine, phenylalanine, leucine, valine, isoleucine and arginine in an amount in the range of ≥ 0.1 wt.% to ≤ 1.0 wt.%,
(E) a cosmetically acceptable aqueous or aqueous-organic medium, and
(J) ethylenediamine-N,N'-disuccinic acid (EDDS), and its salts thereof, in an amount from ≥ 0.20 wt.% to ≤ 2.0 wt.%,
whereby the amount in wt.% is in each case based on the total weight of the oxidative hair care composition.

In an embodiment, the presently claimed invention is directed to an oxidative hair treatment composition comprising
(A) at least one alkalizing agent selected from the group consisting of ammonia, monoethanolamine, propanolamine, isopropanolamine and 2-amino-1-propanol in an amount in the range of ≥ 0.1 wt.% to ≤ 5.0 wt.%,
(B) hydrogen peroxide in an amount in the range of ≥ 0.75 wt.% to ≤ 6.0 wt.%,
(C) squalane in an amount in the range of ≥ 0.2 wt.% to ≤ 3.0 wt.%,
(D) at least one amino acid selected from the group consisting of alanine, glycine, valine, and arginine in an amount in the range of ≥ 0.1 wt.% to ≤ 1.0 wt.%,
(E) a cosmetically acceptable aqueous or aqueous-organic medium, and
(J) ethylenediamine-N,N'-disuccinic acid (EDDS), and its salts thereof, in an amount from ≥ 0.20 wt.% to ≤ 2.0 wt.%,
whereby the amount in wt.% is in each case based on the total weight of the oxidative hair care composition.

Application of the oxidative hair treatment composition to the hair may be undertaken in several ways. Application of the oxidative hair treatment composition may take place on the whole head of hair of an end user. As used herein, the "whole head of hair" means that the hair all over the head from the root of the hair to the tip of the hair is included in the application process. By contrast, the application of the oxidative hair treatment composition may take place only on the root portion of the hair. The application to the root portion of the hair may still be over the entire head of the end user, but application of the oxidative hair treatment composition is applied only to the section of hair closest to the head (root portion), which is between about 0.01 mm to about 40 mm from the scalp of the head. After application to the root portion, product may be applied to the rest of the hair at a later stage to prevent over processing of the hair in the lengths and ends. Also, application may take place on a portion of hair such as the mid of the hair and the tips of the hair. Application of a portion of hair is commonly referred to as highlighting or lowlighting. The portion of hair may be physically separated from the whole head of hair in a hair bundle or may be a smaller portion of hair than the whole head of hair. A hair bundle may be physically separated from a whole head of hair by a device including a plastic cap through which hair bundles are formed when hair is pulled through orifices in the plastic cap, metal foils encompassing a hair bundle, strand separators applied to hair at the root portion, or similar devices.

When present, an optional conditioning agent can be provided in an additional container. In the latter case, the conditioner can be mixed immediately before use and applied together with the other components, or the content of the additional container can be applied (after an optional rinse step) as a post-treatment immediately after the oxidative hair treatment composition.

According to one method for oxidatively colouring hair, the method comprises mixing a tint composition (AA) and an oxidizing composition (BB) and optionally a third component comprising a source of an alkalizing agent such as ammonium persulfate together to form a oxidative hair treatment composition, applying the oxidative hair treatment composition to the hair to form a treated hair surface, waiting for a period of 5-50 minutes, such as 20-35 minutes, and then removing the oxidative hair treatment composition from the treated hair surface through rinsing with water.

The methods of colouring hair also may further comprise working the oxidative hair treatment composition into the treated hair surface by hand or by a tool for a few minutes to ensure uniform application to the entire treated hair surface. The oxidative hair treatment composition remains on the treated hair surface while the end hair colour develops for a time period of 5 to 50 minutes to form oxidatively coloured hair. The consumer then rinses his/her oxidatively coloured hair thoroughly with tap water and allows it to dry and/or styles the oxidatively coloured hair.

In one embodiment of the presently claimed invention, a method of treating hair with the composition preferably comprises the steps of:
a. providing a tint composition (AA) as described herein;
b. providing an oxidizing composition (BB) as described herein;
c. mixing the oxidizing composition (BB) and the tint composition (AA) to obtain a mixed oxidative hair treatment composition as described herein;
d. applying the mixed oxidative hair treatment composition for the oxidative dyeing of keratin fibres onto the hair;
e. leaving the composition on the hair for 5 to 50 minutes; and
f. subsequently rinsing the composition from the hair.

In one embodiment of the presently claimed invention, the oxidative hair treatment composition may be obtained by mixing immediately prior to use a tint composition (AA) and the oxidizing composition (BB). A sufficient amount of the mixed oxidative hair treatment composition is applied to the hair, according to the hair abundance, generally from 20 to 250 grams depending on the amount of hair to be coloured. Upon such preparation, the oxidative hair treatment composition is applied to the hair to be dyed and remains in contact with the hair for an amount of time effective to dye the hair. Typically, the oxidative hair treatment composition is allowed to act on the hair from 5 to 50, preferably 10 to 40, preferably 20 to 35 minutes, at a temperature ranging from 15 to 50 °C. Thereafter, the hair is rinsed with water to remove the composition and dried. If necessary, the hair is washed with a shampoo and rinsed, e.g., with water or a weakly acidic solution, such as a citric acid or tartaric acid solution, and dried. Optionally, another colouring composition or glossing composition can be applied.

The oxidative hair treatment composition can be applied on hair via an applicator bottle or brush. It can be used on full head or partly on single strands (highlight application) as common highlight applicator foils, caps and special applicators can be used, but also freehand techniques such as balayage, with brush and/or combs can be possible. The composition can also be applied as a mousse via a manual spray, a pressurized container or an aerosol mousse. The composition may be dispensed as a solid form to which water is added to generate the oxidant and form a thickened vehicle suitable for hair colouring.

### Embodiments

In the following, there is provided a list of embodiments to further illustrate the present disclosure without intending to limit the disclosure to the specific embodiments listed below.

### Embodiments

1. An oxidative hair treatment composition comprising
   (A) at least one alkalizing agent,
   (B) at least one oxidizing agent,
   (C) at least one saturated acyclic terpane which is a saturated acyclic terpane having 10 to 40 carbon atoms,
   (D) at least one amino acid selected from the group consisting of alanine, glycine, phenylalanine, leucine, valine, isoleucine; glutamic acid, aspartic acid, citrulline, histidine, arginine, lysine and tyrosine,
   (E) a cosmetically acceptable aqueous or aqueous-organic medium, and
   (J) at least one chelant (J) selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, and their salts thereof.
2. The oxidative hair treatment composition according to embodiment 1, wherein the at least one saturated acyclic terpane (C) is a monoterpane, a sesquiterpane, a diterpane, a sesterterpane, a triterpane or a tetraterpane.
3. The oxidative hair treatment composition according to embodiment 1, wherein the at least one saturated acyclic terpane (C) is selected from the group consisting of 2,6-dimethyl octane, 2,6,10,15,19-pentamethylleicosane, farnesane, phytane, squalane, isosqualane, neosqualane and lycopane.
4. The oxidative hair treatment composition according to embodiment 3, wherein the at least one saturated acyclic terpane (C) is selected from the group consisting of squalane, isosqualane and neosqualane.
5. The oxidative hair treatment composition according to embodiment 1, wherein the at least one saturated acyclic terpane (C) has a degree of branching of 2, 3, 4, 5, 6 or 8.
6. The oxidative hair treatment composition according to embodiment 5, wherein the at least one saturated acyclic terpane (C) has a degree of branching of 6.
7. The oxidative hair treatment composition according to any of the preceding embodiments, wherein the at least one saturated acyclic terpane (C) is derived from a bio-based compound.
8. The oxidative hair treatment composition according to embodiment 7, wherein the bio-based compound is selected from the group consisting of squalene and farnesene.
9. The oxidative hair treatment composition according to any of the preceding embodiments, wherein the at least one component (C) is present in an amount in the range of ≥ 0.1 wt.% to ≤ 5.0 wt.%, preferably in the range of ≥ 0.15 wt.% to ≤ 4.0 wt.%, more preferably in the range of ≥ 0.20 wt.% to ≤ 3.5 wt.%, even more preferably in the range of ≥ 0.2 wt.% to ≤ 3.0 wt.%, most preferably in the range of ≥ 0.25 wt.% to ≤ 2.5 wt.%, based on the total weight of the oxidative hair treatment composition.
10. The oxidative hair treatment composition according to any of the preceding embodiments, wherein the at least one amino acid (D) is alanine.
11. The oxidative hair treatment composition according to any of the preceding embodiments, wherein the at least one amino acid (D) is glycine.
12. The oxidative hair treatment composition according to any of the preceding embodiments, wherein the at least one amino acid (D) is valine.
13. The oxidative hair treatment composition according to any of the preceding embodiments, wherein the at least one amino acid (D) is arginine.
14. The oxidative hair treatment composition according to any of the preceding embodiments, wherein the at least one amino acid (D) is obtained from a natural source.
15. The oxidative hair treatment composition according to embodiment 14, wherein the natural source is a plant source.
16. The oxidative hair treatment composition according to any of the preceding embodiments, wherein the at least one component (D) is present in an amount in the range of ≥ 0.05 wt.% to ≤ 2.0 wt.%, preferably in the range of ≥ 0.1 wt.% to ≤ 1.5 wt.%, more preferably in the range of ≥ 0.1 wt.% to ≤ 1.3 wt.%, even more preferably in the range of ≥ 0.1 wt.% to ≤ 1.0 wt.%, based on the total weight of the oxidative hair treatment composition.
17. The oxidative hair treatment composition according to any of the preceding embodiments, wherein the at least one alkalizing agent (A) is selected from the group consisting of ammonia and alkanolamines, or the at least one alkalizing agent (A) is a combination of at least one source of carbonate ions or hydrogen carbonate ions or carbamate ions and at least one source of an alkalizing agent.
18. The oxidative hair treatment according to embodiment 17, wherein the alkanolamines are selected from the group consisting of monoethanolamine, propanolamine, isopropanolamine, 2-amino-2-methyl-1-propanol, 2-amino-1-propanol, 2-amino-1-butanol, 1-amino-2-propanol, 3-amino-1-propanol, 2-aminopropane-1,3-diol, 1-amino-2-butanol, 4-amino-2-butanol, 4-amino-1-butanol, 3-amino-1-butanol, 3-amino-2-butanol, 2-amino-1-butanol, dimethyl glucamine, 2,5-diaminocyclohexane-1,4-diol, 2-aminocycohexan-1-ol, 2,4,6-triaminocyclohexane-1,3,5-triol, 2-amino-2-methyl-1,3-popanediol, 2-amino-1-hydroxylmethyl-1,3-propanediol and 2-dimethylamino-2-methyl-1-propanol.
19. The oxidative hair treatment composition according to embodiment 18, wherein the at least one alkalizing agent (A) is selected from the group consisting of ammonia, monoethanolamine, propanolamine, isopropanolamine and 2-amino-1-propanol.
20. The oxidative hair treatment composition according to embodiment 19, wherein the at least one alkalizing agent (A) is monoethanolamine and/or ammonia.
21. The oxidative hair treatment composition according to embodiment 17, wherein the at least one source of carbonate ions or hydrogen carbonate ions or carbamate ions is selected from the group consisting of sodium, potassium, guanidine, arginine, lithium, calcium, magnesium, barium, ammonium salts of carbonate, carbamate and hydrogen carbonate ions.
22. The oxidative hair treatment composition according to embodiment 17, wherein the at least one source of an alkalizing agent is selected from the group consisting of ammonium chloride, ammonium sulfate, ammonium persulfate, ammonium nitrate, ammonium phosphate, ammonium acetate, ammonium hydroxide and ammonia.
23. The oxidative hair treatment composition according to any of the preceding embodiments, wherein the at least one component (A) is present in an amount in the range of ≥ 0.05 wt.% to ≤ 8.0 wt.%, preferably in an amount in the range of ≥ 0.05 wt.% to ≤ 7.0 wt.%, more preferably in an amount in the range of ≥ 0.1 wt.% to ≤ 6.5 wt.%, most preferably in an amount in the range of ≥ 0.1 wt.% to ≤ 5.0 wt.%, based on the total weight of the oxidative hair treatment composition.
24. The oxidative hair treatment composition according to any of the preceding embodiments, wherein the at least one oxidizing agent (B) is selected from the group consisting of hydrogen peroxide, inorganic alkali metal peroxides, inorganic perhydrate salts and organic peroxides.
25. The oxidative hair treatment composition according to embodiment 24, wherein the at least one oxidizing agent (B) is hydrogen peroxide.
26. The oxidative hair treatment composition according to any of the preceding embodiments, wherein the at least one component (B) is present in an amount in the range of ≥ 0.6 wt.% to ≤ 30.0 wt.%, preferably in an amount in the range from of ≥ 0.7 wt.% to ≤ 25.0 wt.%, more preferably in an amount in the range from of ≥ 0.75 wt.% to ≤ 20.0 wt.%, based on the total weight of the oxidative hair treatment composition.
27. The oxidative hair treatment composition according to any of the preceding embodiments, wherein the cosmetically acceptable aqueous or aqueous-organic medium (E) is present in an amount in the range of ≥ 30.0 to ≤ 95.0 wt.%, based on the total weight of the oxidative hair treatment composition.
28. The oxidative hair treatment composition according to any of the preceding embodiments, wherein the hair treatment composition comprises at least one oxidative primary dye precursor (F) selected from the group consisting of toluene-2,5-diamine, p-phenylenediamine, n-phenyl-p-phenylenediamine, N,N-bis (2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4- amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3- diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof.
29. The oxidative hair treatment composition according to embodiment 28, wherein the at least one oxidative primary dye precursor (F) is selected from the group consisting of toluene-2,5-diamine, hydroxypropyl-p-phenylenediamine, 2-methoxymethyl-p-phenylenediamine and salts thereof.
30. The oxidative hair treatment composition according to any of the preceding embodiments, wherein the oxidative hair treatment composition comprises at least one oxidative coupler dye precursor (G) selected from the group consisting of rescorcinol, 4-chlororesorcinol, 2-chlororesorcinol, 2-methylresorcinol, m-aminophenol, 4- amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotoluene, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4- dimethylpyridine, 2,6-dimethoxy-3,5-pyridinediamine, 3-amino-6-methoxy-2-(methylamino)- pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, resveratrol, isatin, hydroquinone, 4-formyl-1-methylquinolinium-ptoluenesulfonate and salts thereof.
31. The oxidative hair treatment composition according to embodiment 30, wherein at least one oxidative coupler dye precursor (G) is selected from the group consisting of resorcinol, 4-chlororesorcinol, 2-chlororesorcinol, 2-methylresorcinol, m-aminophenol, hydroxybenzomorpholine, 2,4-diaminophenoxyethanol, 2-methyl-5-hydroxyethylaminophenol, 5-((2-hydroxyethyl)amino)-1,3-benzodioxol and salts thereof.
32. The oxidative hair treatment composition according to any of the preceding embodiments, wherein the oxidative hair treatment composition comprises at least one buffering agent (H) which is selected from the group consisting of buffering alkali compounds and buffering acidic compounds.
33. The oxidative hair treatment composition according to embodiment 32, wherein the buffering alkali compounds are selected from the group consisting of alkali metal salts.
34. The oxidative hair treatment composition according to embodiment 32, wherein the buffering acidic compounds are selected from the group consisting of sulfurous acid, sulphuric acid, hydrochloric acid, hyponitrous acid, nitrous acid, nitric acid, phosphoric acid, phosphorous acid, citric acid, and malic acid.
35. The oxidative hair treatment composition according to embodiment 32, wherein the at least one component (H) is selected from the group consisting of disodium phosphate, potassium chloride, phosphoric acid, citric acid, malic acid, hydrochloric acid, hyponitrous acid, and mixtures thereof.
36. The oxidative hair treatment composition according to embodiment 32, wherein the at least one component (H) is citric acid.
37. The oxidative hair treatment composition according to embodiment 32, wherein the at least one component (H) is a mixture of a buffering alkali composition and a buffering acidic composition, wherein the buffering alkali composition is disodium phosphate and the buffering acidic composition is phosphoric acid.
38. The oxidative hair treatment composition according to any one of embodiments 32 to 37, wherein the at least one component (H) is present in an amount in the range of ≥ 0.05 wt.% to ≤ 4.0 wt.%, based on the total weight of the oxidative hair treatment composition.
39. The oxidative hair treatment composition according to any one of the preceding embodiments, wherein the oxidative hair treatment composition has a pH in the range from ≥ 6.0 to ≤ 11.0.
40. The oxidative hair treatment composition according to embodiment 39, wherein the oxidative hair treatment composition has a pH in the range from ≥ 6.5 to ≤ 11.0.
41. The oxidative hair treatment composition according to any of the preceding embodiments, wherein oxidative hair treatment composition comprises at least one direct dye (I) selected from the group consisting of Acid Yellow 1, Disperse Red 17, Basic Brown 17, Acid Black 1, Picramic acid, HC Red 13, N,N'-Bis(2-hydroxyethyl)-2-nitro-p-phenylenediamine, HC Blue 2, HC Yellow 4, HC Yellow 2, HC Orange 1, HC Red 1, HC Red 3, 4-Amino-3-nitrophenol, 3-Nitro-p-hydroxyethylaminophenol, 4-Nitrophenyl aminoethylurea, HC Red 10, HC Red 11, 2-Hydroxyethyl picramic acid, HC Blue 12, Hydroxyethyl-2-nitro-p-toluidine, HC Yellow 12, HC Yellow 7, 2-Chloro-6-(ethylamino)-4-nitrophenol, 2-Amino-6-Chloro-4-Nitrophenol, 4-Hydroxypropylamino-3-nitrophenol, HC Yellow 13, 2,6-Diamino-3-((pyridin-3-yl)azo)pyridine, Basic violet 2, Basic Red 51, HC Blue 16, Basic Orange 1, Basic Red 76, Basic Brown 16, Basic Yellow 57, CI14700, Acid Orange 7, FD&C Yellow 6, Acid Red 33, Acid Yellow 23, Acid Blue 9, Acid violet 9, Acid Red 92, Acid Yellow 3, 1-Amino-4-hydroxy-9,10-anthracendion, CI 60725, Acid violet 43, Disperse Violet 1, Carbon black, Disperse Black 9, Curry red, 2-Hydroxy-1,4-naphthoquinone, Lawsonia Inermis Cera, Indigofera tinctoria, HC Blue 14, CI 42053, Acid Red 52, Acid Green 25, Disperse Blue 377, Pigment Red 57, HC Blue 15, Tetrabromphenol Blue, Anthocyanins, Chlorophyllin-Copper complex, Annatto, Natural Green 3, Betanin, Capsanthin, Basic Yellow 29 and combinations thereof.
42. The oxidative hair treatment composition according to any one of the preceding embodiments, wherein the at least one chelant (J) is ethylenediamine-N,N'-disuccinic acid (EDDS) and its salts.
43. The oxidative hair treatment composition according to any of the preceding embodiments, wherein the at least one chelant (J) is present in an amount from ≥ 0.20 wt.% to ≤ 2.0 wt.%, based on the total weight of the oxidative hair treatment composition.
44. The oxidative hair treatment composition according to any of the preceding embodiments, wherein the oxidative hair treatment composition comprises at least one fatty substance (K) selected from the group consisting of linear or branched, optionally cyclic, C6-C16 alkanes, non-silicone oils of animal, plant, mineral or synthetic origin, fatty alcohols, fatty acids, fatty acid esters and/or fatty alcohol esters, non-silicone waxes, silicones and mixtures thereof.
45. The oxidative hair treatment composition according to embodiment 44, wherein the fatty alcohols are selected from the group consisting of cetyl alcohol, stearyl alcohol, cetostearyl alcohol, cetearyl alcohol, behenyl alcohols, and mixtures thereof.
46. The oxidative hair treatment composition according to embodiment 44 and 45, wherein the at least one component (K) is present in an amount in the range of ≥ 2.0 wt.% to ≤ 70.0 wt.%, based on the total weight of the oxidative hair treatment composition.
47. The oxidative hair treatment composition according to any of the preceding embodiments, wherein the oxidative hair treatment composition comprises at least one thickener (L) selected from the group consisting of polymeric thickeners, mineral thickeners and organic thickeners and gel network thickener systems (L) and/or combinations thereof.
48. The oxidative hair treatment composition according to embodiment 47, wherein the at least one thickener (L) and/or gel network thickener system (L) is present in an amount in the range of ≥ 0.05 wt.% to ≤ 20 wt.%, based on the total weight of the oxidative hair treatment composition.
49. The oxidative hair treatment composition according to any of the preceding embodiments, wherein the oxidative hair treatment composition comprises at least one non-ionic surfactant (M).
50. The oxidative hair treatment composition according to embodiment 49, wherein the at least one component (M) is selected from the group consisting of fatty alcohol polyoxyalkylene esters, alkyl polyoxyalkylene ethers which are derived from C1-C6-alcohols or from C7-C30-fatty alcohols, alkylaryl alcohol polyoxyethylene ethers, alkoxylated animal and/or plant fats and/or oils, glycerol esters, alkylphenol alkoxylates, fatty amine alkoxylates, fatty acid amide and fatty acid diethanolamide alkoxylates, ethoxylates thereof, sugar surfactants, sorbitol esters, polyoxyethylene sorbitan fatty acid esters, alkyl polyglycosides, N-alkylgluconamides, alkyl methyl sulfoxides and alkyl dimethyl phosphine oxides.
51. The oxidative hair treatment composition according to embodiment 50, wherein the alkyl polyoxyalkylene ethers are selected from the group consisting of steareth-20, steareth-100 and steareth-150.
52. A kit comprising;
   (AA) a tint composition comprising components (A), (C), (D) and (J) as defined in any one of the embodiments 1 to 51 and
   (BB) an oxidative composition comprising component (B) and optionally (D) as defined in any one of the embodiments 1 to 51.
53. A method of treating hair with the composition according to any of the preceding embodiments comprises the steps of:
   a. providing a tint composition (AA) comprising components (A) and (J) as defined in any one of the embodiments 1 to 51,
   b. providing an oxidizing composition (BB) comprising component (B) as defined in any one of the embodiments 1 to 51,
      wherein components (C) and (D) as defined in any one of the embodiments 1 to 51 are present in the tint composition (AA) and/or the oxidizing composition (BB), preferably the components (C) and (D) are present in the tint composition and the oxidizing composition optionally comprises the component (D),
   c. mixing the tint composition (AA) and the oxidizing composition (BB) to obtain a hair treatment composition according to any one of the embodiments 1 to 51,
   d. applying the hair treatment composition onto the hair;
   e. leaving the composition on the hair for a period in the range from ≥ 2 to ≤ 50 minutes; and
   f. subsequently rinsing the composition from the hair.
54. The method for treating hair according to embodiment 53, wherein the tint composition (AA) is mixed with the oxidizing composition (BB) in a weight ratio in the range of ≥ 3.0:1.0 to ≤ 1.0:4.0.

### EXAMPLES

The following examples illustrate the formulations and performance results according to the presently claimed invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

### Preparation of hair treatment compositions:

### Test methods

### 1. Hair Damage Measurement via FT-IR

Damage caused to the hair was assessed by a FT-IR (Fourier Transform Infrared) method, which was established to be suitable for studying the effects of keratin surface damage (Strassburger, J., J. Soc. Cosmet Chem., 36, 61 -74 (1985); Joy, M. & Lewis, D.M., Int. J. Cosmet. Sci., 13, 249-261 (1991); Signori, V. and Lewis, D.M., Int. J. Cosmet. Sci., 19, 1-13 (1997)). In particular, these authors have shown that the method was suitable for quantifying the amount of cysteic acid. In general, the oxidation of cystine is thought to be a suitable marker by which to monitor the overall oxidation of the keratinous part of the fibre. Net, the measurement of cysteic acid units by FT-IR was commonly used.

Signori and Lewis (D.M., Int. J. Cosmet. Sci., 19, 1-13 (1997)) showed that FT-IR using a diamond Attenuated Total Internal Reflection (ATR) cell was a sensitive and reproducible way of measuring the cysteic acid content of single fibres and bundles. Hence, the method that was employed to measure the cysteic acid content of multiple fibre bundles and full hair switches, was based upon the FTIR diamond cell ATR method employed by Signori and Lewis (1997). The detailed description of the method for testing the different damage inhibitors follows thereafter:
A Perkin Elmer Spectrum^{®} 1 Fourier Transform Infrared (FTIR) composition equipped with a diamond Attenuated Total Internal Reflection (ATR) cell was used to measure the cysteic acid concentration in mammalian or synthetic hair. In this method, hair switches of various sizes and colours were used. The Treatment Protocol described above was repeated for 3-5 cycles to mimic the behaviour of hair after repeated bleaching cycles. Following this treatment, six readings per switch were taken (1/3, 1/2 and 2/3s down the switch on both sides), and an average calculated. Backgrounds were collected before measurement, and an ATR cell pressure of 1 N/m was employed. A contamination check was performed using the monitor ratio mode of the instrument. As prescribed by Signori and Lewis in 1997, a normalized double derivative analysis routine was used. The original spectra were initially converted to absorbance, before being normalized to the 1450 cm⁻¹ band (the characteristic and invariant protein CH₂ stretch). This normalized absorbance was then twice derivatised using a 13-point averaging. The value of the 1450 cm⁻¹ normalized 2^{nd} derivative of the absorbance at 1040 cm⁻¹ was taken as the relative concentration of cysteic acid. This figure was multiplied by -1×10⁴ to recast it into suitable units.

### 2. Combing Force Measurement

Various external influences, such as certain cosmetic treatments (bleaching, colouring, permanent waving), exposure to the weather, frequent combing and brushing affect the combing ease due to a degradation of the hair cuticle. Thus, the combing ease of hair is a major parameter for describing the quality/degree of damage of hair on the one hand and/or the effectiveness of conditioning respectively hair repair treatments on the other hand. The principle of most methods for the determination of combing properties consists in measuring the force to pull a comb through a hair tress under definite conditions (Ref.: C.R. Robbins, Chemical and Physical Behavior of Human Hair, 5th Edition, p. 646 ff., Springer-Verlag, Berlin Heidelberg 2012. ISBN 978-3-642-25610-3). For this test, an automated device was used in which the tresses to be tested are taken from a storage chamber and are fixed to a force meter. The tresses were combed automatically at constant speed. For each combing stroke the combing force was recorded as a function of the distance combed through (length of the tress). For comparison purposes 20 combing strokes per tress on at least 3 tresses per product sample were averaged. The Wet Combing Force was the average force along the tress. The lower the combing force the better the combing ease.

### 3. Inter Fibre Friction

Friction is the force resisting the movement when a body slides over another one. The cuticle surface has high friction coefficient due to its scale shape and it depends on the cuticle geometry and on the physical-chemical status of the hair. The Inter Fibre Friction method measures the friction between hair fibres of a tress in both directions along the fibre axis by rubbing the dry hair fibres between a skin surrogate at constant pressure. This approach is similar to the characterization of dry hair feel by panellists. The hair interaction was detected by a Texture Analyzer as static friction (peak sum) and dynamic friction (area sum) in both fibre directions. A lower peak sum and lower area sum of friction indicated a higher conditioning performance of hair products (C.R. Robbins, Chemical and Physical Behavior of Human Hair, 5th Edition, p. 622ff., Springer-Verlag, Berlin Heidelberg 2012. ISBN 978-3-642-25610-3)

### Example 1: Hair Damage assessment.

At the first stage, a series of tint compositions with different amino acids were created with the same concentration of the alkalizer in the formula, as shown in Table 1a.

Table 1b shows the oxidative composition in terms of weight percentage of the materials used. Table 1b ingredients were mixed with the tint composition given in Table 1a and provided ready to use oxidative hair colouring formulae.

**Table 1a: Tint compositions with different amino acids.**

| Tint Ingredients | Composition 1A* (%w/w) | Composition 1B (%w/w) | Composition 1C (%w/w) | Composition 1D (%w/w) | Composition 1E (%w/w) |
|---|---|---|---|---|---|
| DI water | q.s | q.s | q.s | q.s | q.s |
| Disodium EDTA (BASF) | 0.100 | 0.100 | 0.100 | 0.100 | 0.100 |
| Ascorbic acid (OSKAR BERG) | 0.300 | 0.300 | 0.300 | 0.300 | 0.300 |
| Sodium Sulfite (BCD CHEMIE) | 0.400 | 0.400 | 0.400 | 0.400 | 0.400 |
| Ammonia Solution (25%) (Brenntag GmbH) | 19.000 | 19.000 | 19.000 | 19.000 | 19.000 |
| Ammonium Sulfate | 2.000 | 2.000 | 2.000 | 2.000 | 2.000 |
| Sodium, Hydroxide | 0.100 | 0.100 | 0.100 | 0.100 | 0.100 |
| Trisodium Ethylenediamine Disuccinate | 3.500 | 3.500 | 3.500 | 3.500 | 3.500 |
| Xanthan Gum | 0.200 | 0.200 | 0.200 | 0.200 | 0.200 |
| Propylene Glycol | 8.000 | 8.000 | 8.000 | 8.000 | 8.000 |
| Dicetyl Phosphate AND Ceteth-10 Phosphate | 10.000 | 10.000 | 10.000 | 10.000 | 10.000 |
| Steareth-20 | 3.000 | 3.000 | 3.000 | 3.000 | 3.000 |
| Alanine | | 2.000 | | | |
| Arginine | | | 2.000 | | |
| Glycine | | | | 2.000 | |
| Valine | | | | | 2.000 |

| | | | | | |
|---|---|---|---|---|---|
| q.s : quantity sufficient * not within the scope of the presently claimed invention | | | | | |

Within the following series of experiments, a series of oxidative hair compositions were tested for their performance for bleaching, lightening or "lifting" pigmented human hair and the associated damage performance.

At the second stage additional 2 tint samples were prepared to evaluate an impact of squalane:

**Table 1b: Tint compositions with different amino acids.**

| Tint Ingredients | Composition 1F (%w/w)* | Composition 1G (%w/w) |
|---|---|---|
| DI water | q.s | q.s |
| Disodium EDTA (BASF) | 0.100 | 0.100 |
| Ascorbic acid (OSKAR BERG) | 0.300 | 0.300 |
| Sodium Sulfite (BCD CHEMIE) | 0.400 | 0.400 |
| Ammonia Solution (25%) (Brenntag GmbH) | 00 | 19.000 |
| Ammonium Sulfate | 2.000 | 2.000 |
| Sodium hydroxide | 0.100 | 0.100 |
| Trisodium Ethylenediamine Disuccinate | 3.500 | 3.500 |
| Xanthan Gum | 0.200 | 0.200 |
| Propylene Glycol | 8.000 | 8.000 |
| Dicetyl Phosphate AND Ceteth-10 Phosphate | 10.000 | 10.000 |
| Steareth-20 | 3.000 | 3.000 |
| Alanine | 1.600 | 1.600 |
| Squalene | | 1.000 |

| | | |
|---|---|---|
| *out of scope of the presently claimed invention | | |

A Minolta spectrophotometer CM-2600d was used to measure the colour of hair tresses using the method described below to determine the L* value to provide a relative measure of lift. A Perkin Elmer Spectrum^{®} 1 Fourier Transform Infrared (FTIR) was equipped with a diamond Attenuated Total Internal Reflection (ATR) cell was used to measure the cysteic acid concentration as a measure of the damage on the hair tress according to the method as described above.

### Hair Damage Performance Testing Method

The tint compositions (1A-1E) were also used within testing to determine the damage performance of each oxidative hair treatment composition.

The following treatment protocol was used:
1. 2 Level 5 hair tresses (Kerling details) were used to test each oxidative hair treatment composition.
2. 1 part of each tint composition (1A to 1E) was mixed with 1 part of bleaching composition containing ammonium persulfate (Blondor^{®} cream) and 2 parts of oxidizing composition (Welloxon^{®} Perfect 6%) and then applied and worked throughout the hair tresses with a brush. 4 g of product was applied to each 1 g tress.
3. The tresses were placed into a 30 °C oven for 30 minutes for the bleaching process to occur.
4. The tresses were then removed from the oven and rinsed in water, 4 L min⁻¹ at a temperature of 37 +- 2 °C for 2 minutes
5. The tresses were then dried with a hair drier.

This procedure was repeated 3 times and the resulting hair tresses were then measured for lift via L*a*b value and damage ATR FT-IR using the methods described above. The lift and hair damage results are captured in the table 2.

**Table 2: Hair Damage Test Results after 3 colouring cycles**

| Example | Tint composition | dFTIR/dL mean | dFTIR/dL Statistical Groups | |
|---|---|---|---|---|
| 1a* | 1A* | 2.497 | A | |
| 1b | 1B | 2.422 | A | B |
| 1c | 1C | 2.415 | A | B |
| 1d | 1D | 2.386 | | B |
| 1e | 1E | 2.383 | | B |

| | | | | |
|---|---|---|---|---|
| * not within the scope of the presently claimed invention | | | | |

1) Student's t test Comparison of all pairs with alpha = 0.05. Letters not connected by the same letter are significantly different.

The results show that the level of damage assessed via ATR FT-IR fell into two statistical groups: examples 1d and 1e show statistically less damage in comparison to example 1a, 1b and 1c show directionally less damage.

At the next stage tint composition 1G and 1F were assessed in terms of their lifting and damage performance by repeating following procedure 5 times.

The tint compositions (1A-1E) were also used within testing to determine the damage performance of each oxidative hair treatment composition.

The following treatment protocol was used:
1. 2 Level 5 hair tresses (Kerling details) were used to test each oxidative hair treatment composition.
2. 1 part of each tint composition (1F to 1G) was mixed with 3 parts of oxidizing composition (Welloxon^{®} Perfect 6%) and then applied and worked throughout the hair tresses with a brush. 4 g of product was applied to each 1 g tress.
3. The tresses were placed into a 30 °C oven for 30 minutes for the bleaching process to occur.
4. The tresses were then removed from the oven and rinsed in water, 4 L min⁻¹ at a temperature of 37 +- 2 °C for 2 minutes
5. The tresses were then dried with a hair drier.

**Table 3: Hair Damage Test Results - part 2**

| Example | Tint composition | DFTIR/dL mean | DFTIR/dL Statistical Groups | |
|---|---|---|---|---|
| 1f* | 1F* | 4.273 | A | |
| 1g | 1G | 3.796 | | B |

| | | | | |
|---|---|---|---|---|
| * not within the scope of the presently claimed invention | | | | |

The results show that hair tresses treated with an oxidative composition containing both alanine and squalane surprisingly had statistically lower level of damage than hair tresses treated with a composition that contained alanine, but no squalane. Thus, the addition of amino acids to the formulation resulted in a lower oxidative damage, which was further lowered with by addition of squalane.

### Example 2: Combing tests

**Table 3: Tint compositions with different oxidative compositions.**

| Tint Ingredients | Composition 2A* (%w/w) | Composition 2B (%w/w) | Composition 2C (%w/w) |
|---|---|---|---|
| DI water | q.s | q.s | q.s |
| Disodium EDTA (BASF) | 0.100 | 0.100 | 0.100 |
| Ascorbic acid (OSKAR BERG) | 0.300 | 0.300 | 0.300 |
| Sodium Sulfite (BCD CHEMIE) | 0.400 | 0.400 | 0.400 |
| Ammonia Solution (25%) (Brenntag GmbH) | 00 | 19.000 | 19.000 |
| Ammonium Sulfate | 2.500 | 2.500 | 2.500 |
| Sodium, Hydroxide | 0.500 | 0.500 | 0.500 |
| Trisodium Ethylenediamine Disuccinate | 3.000 | 3.000 | 3.000 |
| Xanthan Gum | 0.500 | 0.500 | 0.500 |
| Propylene Glycol | 7.000 | 7.000 | 7.000 |
| Cetearyl Alcohol (BASF) | 2.000 | 2.000 | 2.000 |
| Dicetyl Phosphate AND Ceteth-10 Phosphate | 10.000 | 10.000 | 10.000 |
| Steareth-100 | 1.000 | 1.000 | 1.000 |
| Alanine | | 0.250 | 0.500 |
| Squalene | | 1.500 | 2.000 |

| | | | |
|---|---|---|---|
| * not within the scope of the presently claimed invention | | | |

In the examples of the presently claimed invention either Welloxon^{®} Perfect 6% or 9% oxidizing formula (Developer from the Wella Company) was used.

Oxidative compositions produced by mixing tint compositions (2A - 2C) described in Table 3 with a market developer were tested for their impact on hair combability and smoothness.

The following protocol was used to assess the performance of each oxidative hair treatment composition.
1. Three Natural Japanese hair tresses (Kerling) were used to test each oxidative hair treatment composition.
2. Apply a bleaching composition prepared using Welloxon^{®} Perfect 9% and Blondor^{®} to lighten hair to L* = 35 +-2
3. 1 part of each tint composition (2A to 2C) was mixed with 3 parts of oxidizing composition (Illumina^{®} Colour 6%) and then applied and worked throughout the hair tresses with a brush. 4 g of product was applied to each 1 g tress.
3. The tresses were placed into a 30 °C oven for 30 minutes for the bleaching process to occur.
4. The tresses were then removed from the oven and rinsed in water, 4 L min⁻¹ at a temperature of 37 +- 2 °C for 2 minutes
5. The tresses are combed twice (rough & fine side of comb) and placed inside between pre-wettened paper sheets.
6. Combing Force measurements were performed 3 times for each tress.

The measurement results are presented in Table 4.

**Table 4: Combing Force measurements**

| Example | Tint composition used to make oxidative mixture | Combing Force Average, N | Combing Force Standard deviation, N | Statistical Groups' | |
|---|---|---|---|---|---|
| 2a* | 2A* | 0.407 | 0.057 | A | |
| 2b | 2B | 0.293 | 0.027 | | B |
| 2c | 2C | 0.283 | 0.029 | | B |

| | | | | | |
|---|---|---|---|---|---|
| * not within the scope of the presently claimed invention 3) T-test Comparison of all pairs with significance >=95%. Letters not connected by the same letter are significantly different. | | | | | |

Results show that tresses treated with oxidative compositions based on 2B and 2C mixed with Illumina^{®} Colour Developer 6% required statistically significantly less force to comb through than the ones treated with the oxidative composition 2A and the same developer. It showed that the addition of squalane and alanine to the formulation improved hair smoothness, which can reduce mechanical damage when combing hair.

### Example 3: Hair Softness tests

Two of the tint compositions (2A and 2C) were selected to prepare oxidative mixtures by mixing with 6% developer to be used for inter fibre friction measurements.

The following protocol was used to assess the performance of prepared oxidative hair treatment composition.
1. Three Natural Japanese hair tresses (Kerling details) were used to test each oxidative hair treatment composition .
2. Apply a bleaching composition prepared using Welloxon^{®} Perfect 9% and Blondor^{®} to lighten hair to L* = 35 +-2
3. 1 part of each tint composition (2A to 2C) was mixed with 3 parts of oxidizing composition (Illumina Colour 6%) and then applied and worked throughout the hair tresses with a brush. 4 g of product was applied to each 1 g tress.
3. The tresses were placed into a 30 °C oven for 30 minutes for the bleaching process to occur.
4. The tresses were then removed from the oven and rinsed in water, 4 L min⁻¹ at a temperature of 37 +- 2 °C for 2 minutes. The tresses are adjusted to 50% rel. humidity by weight.
5. The tresses are combed, placed on a tray and then in a climate chamber to avoid the impact of difference in humidity and temperature.
6. Measurements performed by rubbing hair fibres between a skin surrogate in an upward and downward direction, so that the hair interaction is detected.

The measurement results are presented in Table 5.

**Table 5: Inter Fibre Friction measurement results**

| Example | Tint used to make oxidative mixture | Friction, average | Friction, stddev | Statistical Groups' | |
|---|---|---|---|---|---|
| 3a* | 2A* | 7672 | 382 | A | |
| 3c | 2C | 6819 | 205 | | B |

| | | | | | |
|---|---|---|---|---|---|
| * not within the scope of the presently claimed invention 3) T-test Comparison of all pairs with significance >=95%. Letters not connected by the same letter are significantly different. | | | | | |

Measurement results show statistically significant differences between the tested oxidative compositions. Lower inter fibre friction is indicative of better conditioning properties, that are often perceived as softer hair feel. Thus, oxidative composition containing both amino acids and squalane delivered softer/ better hair feel.

### Example 4: Sensorial assessment.

A panel of 12 individuals were asked to assess tresses treated with oxidative compositions obtained by mixing oxidative tints described below with Welloxon developer 6%.

**Table 6**

| Tint Ingredients | Composition 3A* | Composition 3B | Composition 3C * |
|---|---|---|---|
| | (%w/w) | (%w/w) | (%w/w) |
| DI water | q.s | q.s | q.s |
| Disodium EDTA (BASF) | 0.100 | 0.100 | 0.100 |
| Ascorbic acid (OSKAR BERG) | 0.300 | 0.300 | 0.300 |
| Sodium Sulfite (BCD CHEMIE) | 0.400 | 0.400 | 0.400 |
| Ammonia Solution (25%) (Brenntag GmbH) | 18.000 | 18.000 | 18.000 |
| Ammonium Sulfate | 2.000 | 2.000 | 2.000 |
| Sodium, Hydroxide | 0.500 | 0.500 | 0.500 |
| 1-Hydroxyethyl 4,5-Diamino Pyrazole Sulfate | 0.005 | 0.005 | 0.005 |
| 2-Methoxymethyl-p-Phenylenediamine | 0.225 | 0.225 | 0.225 |
| Resorcinol | 0.075 | 0.075 | 0.075 |
| Hydroxyethyl-3,4-Methylenedioxyaniline HCl | 0.09 | 0.09 | 0.09 |
| 2,4-Diaminophenoxyethanol HCl | 0.11 | 0.11 | 0.11 |
| 4-Amino-2-Hydroxytoluene | 0.003 | 0.003 | 0.003 |
| Trisodium Ethylenediamine Disuccinate | 2.500 | 2.500 | 2.500 |
| Xanthan Gum | 0.250 | 0.250 | 0.250 |
| Propylene Glycol | 7.000 | 7.000 | 7.000 |
| Cetearyl Alcohol (BASF) | 9.000 | 9.000 | 9.000 |
| Dicetyl Phosphate AND Ceteth-10 Phosphate | 2.000 | 2.000 | 2.000 |
| Steareth-200 | 1.250 | 1.250 | 1.250 |
| Alanine | | 0.19 | 0.19 |
| Valine | | 0.19 | 0.19 |
| Arginine | | 0.19 | 0.19 |
| Glycine | | 0.19 | 0.19 |
| Squalane | | 3.00 | |

| | | | |
|---|---|---|---|
| * not within the scope of the presently claimed invention | | | |

The tint compositions (3A - 3C) were used to prepare oxidative compositions.

The following treatment protocol was used:
1. 2 Level 4 hair tresses (Kerling details) were used to test each oxidative hair treatment composition .
2. 1 part of each tint composition (3A-3C) was mixed with 2 parts of oxidizing composition (Welloxon^{®} Perfect 6%) and then applied and worked throughout the hair tresses with a brush. 4 g of product was applied to each 1 g tress.
3. The tresses were placed into a 30 °C oven for 30 minutes for colouring to occur.
4. The tresses were then removed from the oven and rinsed in water, 4 L min⁻¹ at a temperature of 37 +- 2 °C for 2 minutes
5. The tresses were then dried with a hair drier.

Panelists were given 3 tresses to assess hair smoothness by assigning a value of 1, if any of the tresses had a superior feel or 0, if no differences were detected. Results show that tresses treated with the composition made by mixing 3B with Welloxon^{®} Developer 6% were assessed as the smoothest one with additional comments from panelists about more conditioned hair feel.

**Table 7**

| Example | Oxidative tint in mixture | Average score |
|---|---|---|
| 4a* | 3A* | 0.083 |
| 4b | 3B | 0.750 |
| 4c* | 3C* | 0.166 |

| | | |
|---|---|---|
| * not within the scope of the presently claimed invention | | |

## Claims

1. An oxidative hair treatment composition comprising
(A) at least one alkalizing agent,
(B) at least one oxidizing agent,
(C) at least one saturated acyclic terpane which is a saturated acyclic terpane having 10 to 40 carbon atoms,
(D) at least one amino acid selected from the group consisting of alanine, glycine, phenylalanine, leucine, valine, isoleucine, glutamic acid, aspartic acid, citrulline, histidine, arginine, lysine and tyrosine,
(E) a cosmetically acceptable aqueous or aqueous-organic medium, and
(J) at least one chelant (J) selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, and their salts thereof.

2. The oxidative hair treatment composition according to claim 1, wherein the at least one saturated acyclic terpane (C) is selected from the group consisting of 2,6-dimethyl octane, 2,6,10,15,19-pentamethylleicosane, farnesane, phytane, squalane, isosqualane, neosqualane and lycopane.

3. The oxidative hair treatment composition according to claim 2, wherein the at least one saturated acyclic terpane (C) is squalane.

4. The oxidative hair treatment composition according to any of the preceding claims,
wherein the at least one component (C) is present in an amount in the range of ≥ 0.1 wt.% to ≤ 5.0 wt.%, preferably in the range of ≥ 0.15 wt.% to ≤ 4.0 wt.%, more preferably in the range of ≥ 0.20 wt.% to ≤ 3.5 wt.%, even more preferably in the range of ≥ 0.2 wt.% to ≤ 3.0 wt.%, most preferably in the range of ≥ 0.25 wt.% to ≤ 2.5 wt.%, based on the total weight of the oxidative hair treatment composition.

5. The oxidative hair treatment composition according to any of the preceding claims, wherein the at least one amino acid (D) is alanine.

6. The oxidative hair treatment composition according to any of the preceding claims, wherein the at least one amino acid (D) is glycine.

7. The oxidative hair treatment composition according to any of the preceding claims, wherein the at least one amino acid (D) is valine.

8. The oxidative hair treatment composition according to any of the preceding claims, wherein the at least one amino acid (D) is arginine.

9. The oxidative hair treatment composition according to any of the preceding claims,
wherein the at least one component (D) is present in an amount in the range of ≥ 0.05 wt.% to ≤ 2.0 wt.%, preferably in the range of ≥ 0.1 wt.% to ≤ 1.5 wt.%, more preferably in the range of ≥ 0.1 wt.% to ≤ 1.3 wt.%, even more preferably in the range of ≥ 0.1 wt.% to ≤ 1.0 wt.%, based on the total weight of the oxidative hair treatment composition.

10. The oxidative hair treatment composition according to any of the preceding claims,
wherein the at least one alkalizing agent (A) is selected from the group consisting of ammonia and alkanolamines, or the at least one alkalizing agent (A) is a combination of at least one source of carbonate ions or hydrogen carbonate ions or carbamate ions and at least one source of an alkalizing agent.

11. The oxidative hair treatment composition according to any of the preceding claims,
wherein the at least one component (A) is present in an amount in the range of ≥ 0.05 wt.% to ≤ 8.0 wt.%, preferably in an amount in the range of ≥ 0.05 wt.% to ≤ 7.0 wt.%, more preferably in an amount in the range of ≥ 0.1 wt.% to ≤ 6.5 wt.%, most preferably in an amount in the range of ≥ 0.1 wt.% to ≤ 5.0 wt.%, based on the total weight of the oxidative hair treatment composition.

12. The oxidative hair treatment composition according to any of the preceding claims, wherein the at least one oxidizing agent (B) is hydrogen peroxide.

13. The oxidative hair treatment composition according to any of the preceding claims,
wherein the at least one component (B) is present in an amount in the range of ≥ 0.6 wt.% to ≤ 30.0 wt.%, preferably in an amount in the range from of ≥ 0.7 wt.% to ≤ 25.0 wt.%, more preferably in an amount in the range from of ≥ 0.75 wt.% to ≤ 20.0 wt.%, based on the total weight of the oxidative hair treatment composition.

14. The oxidative hair treatment composition according to any of the preceding claims,
wherein the cosmetically acceptable aqueous or aqueous-organic medium (E) is present in an amount in the range of ≥ 30.0 to ≤ 95.0 wt.%, based on the total weight of the oxidative hair treatment composition.

15. The oxidative hair treatment composition according to any one of the preceding claims, wherein the oxidative hair treatment composition has a pH in the range from ≥ 6.0 to ≤ 11.0.

16. The oxidative hair treatment composition according to any one of the preceding claims, wherein the at least one chelant (J) is ethylenediamine-N,N'-disuccinic acid (EDDS) and its salts.

17. The oxidative hair treatment composition according to any of the preceding claims,
wherein the at least one chelant (J) is present in an amount from ≥ 0.20 wt.% to ≤ 2.0 wt.%, based on the total weight of the oxidative hair treatment composition.

18. A kit comprising;
(AA) a tint composition comprising components (A), (C), (D) and (J) as defined in any one of the claims 1 to 17 and
(BB) an oxidative composition comprising component (B) and optionally (D) as defined in any one of the claims 1 to 17.

19. A method of treating hair with the composition according to any of the preceding claims comprises the steps of:
a. providing a tint composition (AA) comprising components (A) and (J) as defined in any one of the claims 1 to 17,
b. providing an oxidizing composition (BB) comprising component (B) as defined in any one of the claims 1 to 17,
wherein components (C) and (D) as defined in any one of the claims 1 to 17 are present in the tint composition (AA) and/or the oxidizing composition (BB),
c. mixing the tint composition (AA) and the oxidizing composition (BB) to obtain a hair treatment composition according to any one of the claims 1 to 17,
d. applying the hair treatment composition onto the hair;
e. leaving the composition on the hair for a period in the range from ≥ 2 to ≤ 50 minutes; and
f. subsequently rinsing the composition from the hair.

## Patentansprüche

1. Oxidative Haarbehandlungszusammensetzung, umfassend
(A) mindestens ein Alkalisierungsmittel,
(B) mindestens ein Oxidationsmittel,
(C) mindestens ein gesättigtes azyklisches Terpan, das ein gesättigtes azyklisches Terpan ist, das 10 bis 40 Kohlenstoffatome aufweist,
(D) mindestens eine Aminosäure, ausgewählt aus der Gruppe, bestehend aus Alanin, Glycin, Phenylalanin, Leucin, Valin, Isoleucin, Glutaminsäure, Asparaginsäure, Citrullin, Histidin, Arginin, Lysin und Tyrosin,
(E) ein kosmetisch annehmbares wässriges oder wässrig-organisches Medium, und
(J) mindestens einen Chelatbildner (J), ausgewählt aus der Gruppe, bestehend aus Ethylendiamin-N,N'-dibernsteinsäure (EDDS), Ethylendiamin-N,N'-diglutarsäure (EDDG), 2-Hydroxypropylendiamin-N,N'-dibernsteinsäure (HPDDS), N,N-Dicarboxymethylglutaminsäure (GLDA), Glycinamid-N,N'-bernsteinsäure (GADS), Ethylendiamin-N-N'-bis(ortho-Hydroxyphenylessigsäure) (EDDHA), Dimethylglucamin (DMG), N-(1-Carboxyethyl)iminodiessigsäure, Methylglycin-N,N-diessigsäure und ihre Salze davon.

2. Oxidative Haarbehandlungszusammensetzung nach Anspruch 1, wobei das mindestens eine gesättigte azyklische Terpan (C) ausgewählt ist aus der Gruppe, bestehend aus 2,6-Dimethyloctan, 2,6,10,15,19-Pentamethylleicosan, Farnesan, Phytan, Squalan, Isosqualan, Neosqualan und Lycopan.

3. Oxidative Haarbehandlungszusammensetzung nach Anspruch 2, wobei das mindestens eine gesättigte azyklische Terpan (C) Squalan ist.

4. Oxidative Haarbehandlungszusammensetzung nach einem der vorherigen Ansprüche, wobei die mindestens eine Komponente (C) in einer Menge in dem Bereich von ≥ 0,1 Gewichts-% bis ≤ 5,0 Gewichts-%, vorzugsweise in dem Bereich von ≥ 0,15 Gewichts-% bis ≤ 4,0 Gewichts-%, bevorzugter in dem Bereich von ≥ 0,20 Gewichts-% bis ≤ 3,5 Gewichts-%, noch bevorzugter in dem Bereich von ≥ 0,2 Gewichts-% bis ≤ 3,0 Gewichts-%, am meisten bevorzugt in dem Bereich von ≥ 0,25 Gewichts-% bis ≤ 2,5 Gewichts-%, bezogen auf das Gesamtgewicht des oxidativen Haarbehandlungsmittels vorhanden ist.

5. Oxidative Haarbehandlungszusammensetzung nach einem der vorherigen Ansprüche, wobei die mindestens eine Aminosäure (D) Alanin ist.

6. Oxidative Haarbehandlungszusammensetzung nach einem der vorherigen Ansprüche, wobei die mindestens eine Aminosäure (D) Glycin ist.

7. Oxidative Haarbehandlungszusammensetzung nach einem der vorherigen Ansprüche, wobei die mindestens eine Aminosäure (D) Valin ist.

8. Oxidative Haarbehandlungszusammensetzung nach einem der vorherigen Ansprüche, wobei die mindestens eine Aminosäure (D) Arginin ist.

9. Oxidative Haarbehandlungszusammensetzung nach einem der vorherigen Ansprüche, wobei die mindestens eine Komponente (D) in einer Menge in dem Bereich von ≥ 0,05 Gewichts-% bis ≤ 2,0 Gewichts-%, vorzugsweise in dem Bereich von ≥ 0,1 Gewichts-% bis ≤ 1,5 Gewichts-%, bevorzugter in dem Bereich von ≥ 0,1 Gewichts-% bis ≤ 1,3 Gewichts-%, noch bevorzugter in dem Bereich von ≥ 0,1 Gewichts-% bis ≤ 1,0 Gewichts-%, bezogen auf das Gesamtgewicht des oxidativen Haarbehandlungsmittels, vorhanden ist.

10. Oxidative Haarbehandlungszusammensetzung nach einem der vorherigen Ansprüche, wobei das mindestens eine Alkalisierungsmittel (A) ausgewählt ist aus der Gruppe, bestehend aus Ammoniak und Alkanolaminen, oder das mindestens eine Alkalisierungsmittel (A) eine Kombination aus mindestens einer Quelle von Carbonationen oder Hydrogencarbonationen oder Carbamationen und mindestens einer Quelle eines Alkalisierungsmittels ist.

11. Oxidative Haarbehandlungszusammensetzung nach einem der vorherigen Ansprüche, wobei die mindestens eine Komponente (A) in einer Menge in dem Bereich von ≥ 0,05 Gewichts-% bis ≤ 8,0 Gewichts-%, vorzugsweise in einer Menge in dem Bereich von ≥ 0,05 Gewichts-% bis ≤ 7,0 Gewichts-%, bevorzugter in einer Menge in dem Bereich von ≥ 0,1 Gewichts-% bis ≤ 6,5 Gewichts-%, am meisten bevorzugt in einer Menge in dem Bereich von ≥ 0,1 Gewichts-% bis ≤ 5,0 Gewichts-%, bezogen auf das Gesamtgewicht des oxidativen Haarbehandlungsmittels, vorhanden ist.

12. Oxidative Haarbehandlungszusammensetzung nach einem der vorherigen Ansprüche, wobei das mindestens eine Oxidationsmittel (B) Wasserstoffperoxid ist.

13. Oxidative Haarbehandlungszusammensetzung nach einem der vorherigen Ansprüche, wobei die mindestens eine Komponente (B) in einer Menge in dem Bereich von ≥ 0,6 Gewichts-% bis ≤ 30,0 Gewichts-%, vorzugsweise in einer Menge in dem Bereich von ≥ 0,7 Gewichts-% bis ≤ 25,0 Gewichts-%, bevorzugter in einer Menge in dem Bereich von ≥ 0,75 Gewichts-% bis ≤ 20,0 Gewichts-%, bezogen auf das Gesamtgewicht der oxidativen Haarbehandlungszusammensetzung, vorhanden ist.

14. Oxidative Haarbehandlungszusammensetzung nach einem der vorherigen Ansprüche, wobei das kosmetisch annehmbare wässrige oder wässrig-organische Medium (E) in einer Menge in dem Bereich von ≥ 30,0 bis ≤ 95,0 Gewichts-%, bezogen auf das Gesamtgewicht der oxidativen Haarbehandlungszusammensetzung, vorhanden ist.

15. Oxidative Haarbehandlungszusammensetzung nach einem der vorherigen Ansprüche, wobei die oxidative Haarbehandlungszusammensetzung einen pH in dem Bereich von ≥ 6,0 bis ≤ 11,0 aufweist.

16. Oxidative Haarbehandlungszusammensetzung nach einem der vorherigen Ansprüche, wobei der mindestens eine Chelatbildner (J) Ethylendiamin-N,N'-dibernsteinsäure (EDDS) und ihre Salze ist.

17. Oxidative Haarbehandlungszusammensetzung nach einem der vorherigen Ansprüche, wobei der mindestens eine Chelatbildner (J) in einer Menge von ≥ 0,20 Gewichts-% bis ≤ 2,0 Gewichts-%, bezogen auf das Gesamtgewicht der oxidativen Haarbehandlungszusammensetzung, vorhanden ist.

18. Kit, umfassend:
(AA) eine Färbezusammensetzung, umfassend Komponenten (A), (C), (D) und (J), wie definiert in einem der Ansprüche 1 bis 17, und
(BB) eine oxidative Zusammensetzung, umfassend die Komponente (B) und optional (D), wie definiert in einem der Ansprüche 1 bis 17.

19. Verfahren zum Behandeln von Haar mit der Zusammensetzung nach einem der vorherigen Ansprüche, umfassend die folgenden Schritte:
a. Bereitstellen einer Färbezusammensetzung (AA), umfassend Komponenten (A) und (J) wie definiert in einem der Ansprüche 1 bis 17,
b. Bereitstellen einer oxidierenden Zusammensetzung (BB), umfassend Komponente (B) wie definiert in einem der Ansprüche 1 bis 17,
wobei Komponente (C) und (D) wie definiert in einem der Ansprüche 1 bis 17 in der Färbezusammensetzung (AA) und/oder der Oxidationszusammensetzung (BB) vorhanden sind,
c. Mischen der Färbezusammensetzung (AA) und der oxidierenden Zusammensetzung (BB), um eine Haarbehandlungszusammensetzung nach einem der Ansprüche 1 bis 17 zu erlangen,
d. Auftragen der Haarbehandlungszusammensetzung auf das Haar;
e. Belassen der Zusammensetzung auf dem Haar über einen Zeitraum in dem Bereich von ≥ 2 bis ≤ 50 Minuten; und
f. anschließen Spülen der Zusammensetzung aus dem Haar.

## Revendications

1. Composition de traitement capillaire oxydante comprenant
(A) au moins un agent alcalinisant,
(B) au moins un agent oxydant,
(C) au moins un terpane acyclique saturé qui est un terpane acyclique saturé ayant 10 à 40 atomes de carbone,
(D) au moins un acide aminé choisi dans le groupe constitué par l'alanine, la glycine, la phénylalanine, la leucine, la valine, l'isoleucine, l'acide glutamique, l'acide aspartique, la citrulline, l'histidine, l'arginine, la lysine et la tyrosine,
(E) un milieu aqueux ou aqueux-organique cosmétiquement acceptable, et
(J) au moins un chélateur (J) choisi dans le groupe constitué par l'acide éthylènediamine-N,N'-disuccinique (EDDS), l'acide éthylènediamine-N,N'-diglutarique (EDDG), l'acide 2-hydroxypropylènediamine-N,N'-disuccinique (HPDDS), l'acide N,N-dicarboxyméthylglutamique (GLDA), l'acide glycinamide-N,N'-disuccinique (GADS), l'acide éthylènediamine-N-N'-bis(ortho-hydroxyphénylacétique) (EDDHA), la diméthylglucamine (DMG), l'acide N-(1-carboxyéthyl)iminodiacétique, l'acide méthylglycine-N,N-diacétique et des sels de ceux-ci.

2. Composition de traitement capillaire oxydante selon la revendication 1, dans laquelle le ou les terpanes acycliques saturés (C) sont choisis dans le groupe constitué par le 2,6-diméthyloctane, le 2,6,10,15,19-pentaméthylléicosane, le farnésane, le phytane, le squalane, l'isosqualane, le néosqualane et le lycopane.

3. Composition de traitement capillaire oxydante selon la revendication 2, dans laquelle le ou les terpanes acycliques saturés (C) sont le squalane.

4. Composition de traitement capillaire oxydante selon l'une quelconque des revendications précédentes, dans laquelle le ou les composants (C) sont présents en une quantité dans la plage de ≥ 0,1 % en poids à ≤ 5,0 % en poids, de préférence dans la plage de ≥ 0,15 % en poids à ≤ 4,0 % en poids, plus préférablement dans la plage de ≥ 0,20 % en poids à ≤ 3,5 % en poids, encore plus préférablement dans la plage de ≥ 0,2 % en poids à ≤ 3,0 % en poids, de manière préférée entre toutes dans la plage de ≥ 0,25 % en poids à ≤ 2,5 % en poids, par rapport au poids total de la composition de traitement capillaire oxydante.

5. Composition de traitement capillaire oxydante selon l'une quelconque des revendications précédentes, dans laquelle le ou les acides aminés (D) sont l'alanine.

6. Composition de traitement capillaire oxydante selon l'une quelconque des revendications précédentes, dans laquelle le ou les acides aminés (D) sont la glycine.

7. Composition de traitement capillaire oxydante selon l'une quelconque des revendications précédentes, dans laquelle le ou les acides aminés (D) sont la valine.

8. Composition de traitement capillaire oxydante selon l'une quelconque des revendications précédentes, dans laquelle le ou les acides aminés (D) sont l'arginine.

9. Composition de traitement capillaire oxydante selon l'une quelconque des revendications précédentes, dans laquelle le ou les composants (D) sont présents en une quantité dans la plage de ≥ 0,05 % en poids à ≤ 2,0 % en poids, de préférence dans la plage de ≥ 0,1 % en poids à ≤ 1,5 % en poids, plus préférablement dans la plage de ≥ 0,1 % en poids à ≤ 1,3 % en poids, encore plus préférablement dans la plage de ≥ 0,1 % en poids à ≤ 1,0 % en poids, par rapport au poids total de la composition de traitement capillaire oxydante.

10. Composition de traitement capillaire oxydante selon l'une quelconque des revendications précédentes, dans laquelle le ou les agents alcalinisants (A) sont choisis dans le groupe constitué par l'ammoniac et les alcanolamines, ou le ou les agents alcalinisants (A) sont une combinaison d'au moins une source d'ions carbonate ou d'ions hydrogénocarbonate ou d'ions carbamate et d'au moins une source d'un agent alcalinisant.

11. Composition de traitement capillaire oxydante selon l'une quelconque des revendications précédentes, dans laquelle le ou les composants (A) sont présents en une quantité dans la plage de ≥ 0,05 % en poids à ≤ 8,0 % en poids, de préférence en une quantité dans la plage de ≥ 0,05 % en poids à ≤ 7,0 % en poids, plus préférablement en une quantité dans la plage de ≥ 0,1 % en poids à ≤ 6,5 % en poids, de manière préférée entre toutes en une quantité dans la plage de ≥ 0,1 % en poids à ≤ 5,0 % en poids, par rapport au poids total de la composition de traitement capillaire oxydante.

12. Composition de traitement capillaire oxydante selon l'une quelconque des revendications précédentes, dans laquelle le ou les agents oxydants (B) sont le peroxyde d'hydrogène.

13. Composition de traitement capillaire oxydante selon l'une quelconque des revendications précédentes, dans laquelle le ou les composants (B) sont présents en une quantité dans la plage de ≥ 0,6 % en poids à ≤ 30,0 % en poids, de préférence en une quantité dans la plage de ≥ 0,7 % en poids à ≤ 25,0 % en poids, plus préférablement en une quantité dans la plage de ≥ 0,75 % en poids à ≤ 20,0 % en poids, par rapport au poids total de la composition de traitement capillaire oxydante.

14. Composition de traitement capillaire oxydante selon l'une quelconque des revendications précédentes, dans laquelle le milieu aqueux ou aqueux-organique cosmétiquement acceptable (E) est présent en une quantité dans la plage de ≥ 30,0 à ≤ 95,0 % en poids, par rapport au poids total de la composition de traitement capillaire oxydante.

15. Composition de traitement capillaire oxydante selon l'une quelconque des revendications précédentes, la composition de traitement capillaire oxydante ayant un pH dans la plage de ≥ 6,0 à ≤ 11,0.

16. Composition de traitement capillaire oxydante selon l'une quelconque des revendications précédentes, dans laquelle le ou les chélateurs (J) sont l'acide éthylènediamine-N,N'-disuccinique (EDDS) et ses sels.

17. Composition de traitement capillaire oxydante selon l'une quelconque des revendications précédentes, dans laquelle le ou les chélateurs (J) sont présents en une quantité de ≥ 0,20 % en poids à ≤ 2,0 % en poids, par rapport au poids total de la composition de traitement capillaire oxydante.

18. Trousse comprenant :
(AA) une composition de teinture comprenant les composants (A), (C), (D) et (J) tels que définis dans l'une quelconque des revendications 1 à 17 et
(BB) une composition oxydante comprenant le composant (B) et éventuellement (D) tel que défini dans l'une quelconque des revendications 1 à 17.

19. Procédé de traitement capillaire avec la composition selon l'une quelconque des revendications précédentes comprenant les étapes de :
a. fourniture d'une composition de teinture (AA) comprenant les composants (A) et (J) tels que définis dans l'une quelconque des revendications 1 à 17,
b. fourniture d'une composition oxydante (BB) comprenant le composant (B) tel que défini dans l'une quelconque des revendications 1 à 17,
dans lequel les composants (C) et (D) tels que définis dans l'une quelconque des revendications 1 à 17 sont présents dans la composition de teinture (AA) et/ou la composition oxydante (BB),
c. mélange de la composition de teinture (AA) et de la composition oxydante (BB) pour obtenir une composition de traitement capillaire selon l'une quelconque des revendications 1 à 17,
d. application de la composition de traitement capillaire sur les cheveux ;
e. maintien de la composition sur les cheveux pendant une durée dans la plage de ≥ 2 à ≤ 50 minutes ; et
f. ensuite rinçage de la composition à partir des cheveux.
